# EUROPEAN PATENT APPLICATION

(11) **EP 4 659 764 A1**
(43) Date of publication of application: **10.12.2025**
(21) Application number: 24749655.7
(22) Date of filing: 29.01.2024
(51) Int. Cl.: A61K 47/68, C07D 207/452, C07C 33/16, C07D 495/22, A61P 35/00

(54) **LINKER DRUG, AND PREPARATION METHOD AND USE OF ANTIBODY-DRUG CONJUGATE THEREOF**

(30) Priority: 31.01.2023 CN 202310093383; 28.07.2023 CN 202310947390
(71) Applicant: Lepu Biopharma Co., Ltd., Shanghai 201114 (CN)
(72) Inventor: LI, Hongfeng, Shanghai 201203 (CN); LIU, Wei, Shanghai 201203 (CN); MA, Tian, Shanghai 201203 (CN); LIU, Wenchao, Shanghai 201203 (CN); WU, Xiangyu, Shanghai 201203 (CN); WANG, Yanchun, Shanghai 201203 (CN); XU, Zhenyi, Shanghai 201203 (CN); HU, Chaohong, Shanghai 201114 (CN); QIN, Minmin, Shanghai 201114 (CN)
(74) Representative: Mewburn Ellis LLP
(86) International application number: PCT/CN2024/074508
(87) International publication number: WO 2024/160176

(57) **Abstract**

A linker-drug conjugate, an antibody-drug conjugate, and a preparation method therefor and the use thereof. The linker-drug conjugate has a structure as shown in formula I, i.e., M-X-D (formula I), wherein M is a chemical structure comprising a maleimide (m) fragment or a cyclooctyne fragment, X is a linker fragment composed of 1-5 amino acids or derivatives thereof, and D is a cytotoxin. The linker-drug conjugate can release Payload by means of an enzymatic hydrolysis reaction in the case of removal of hydrophobic structure PAB, which can not only reduce the hydrophobicity of the Linker-Drug and reduce the content of a polymer produced in the preparation of ADC, but also can exhibit the corresponding cell killing activity.

## Description

### Cross-Reference to Related Applications

The present application is based on and claims the priority to the application with the CN application number of 202310093383.0 and the filing date of January 31, 2023, as well as the application with the CN application number of 202310947390.2 and the filing date of July 28, 2023, the contents of both of which are hereby incorporated by reference into the present application in their entirety.

### Technical Field

The present invention relates to the pharmaceutical field, and specifically relates to a linker-drug conjugate, an antibody-drug conjugate, and a preparation method therefor and the use thereof.

### Background Art

The research on antibody-drug conjugates (ADCs) can be traced back to the 1980s. However, it was not until 2000 that the first antibody-drug conjugate (trade name: Mylotarg, developed by Pfizer) was approved by the FDA for the treatment of acute granulocytic leukemia. Due to limitations in conjugation technology, targeting ability, effectiveness, etc., the intact antibody-drug conjugate was unstable in the blood, resulting in the development of lethal toxicity, and it was withdrawn from the market in 2010. This has cast an even darker shadow over the already uncertain research on ADC drugs.

However, through improvements to the pre-existing technology and by using its own novel antibody conjugation technology, Seagen developed a novel antibody-drug conjugate, brentuximab vedotin (SGN-35, trade name: Adcetris), which was already approved by the FDA in 2011 for the treatment of Hodgkin lymphoma and systemic anaplastic large cell lymphoma. In 2013, there was another breakthrough in antibody-drug conjugates. Ado-trastuzumab emtansine (T-DM1, trade name: Kadcyla), the first antibody-drug conjugate targeting solid tumors, jointly developed by Genentech and ImmunoGen, was approved by the FDA for the treatment of HER2-positive breast cancer. With the successful development of these two drugs, ADC drugs have once again entered the research spotlight of people in a booming state.

In recent years, ADCs have developed rapidly. Currently, 15 ADC drugs have been approved for marketing, and more than 200 ADCs have entered clinical trials. ADCs are playing an increasingly important role in the field of tumor targeted therapy.

An antibody-drug conjugate consists of three parts: an antibody (Ab), a linker, and a small-molecule cytotoxic drug (payload/warhead). The antibody part of an ADC should possess tumor specificity, good endocytic efficiency, good antigen affinity, and no or low immunogenicity. IgG1 is the most commonly used antibody part. The warhead part is typically a cytotoxic drug that acts on microtubules, DNA, or RNA, such as camptothecins, maytansines, and auristatins, which needs to be sufficiently toxic and sufficiently water-soluble, and its target(s) of action is inside the cells. The linker that links the antibody and the small-molecule cytotoxic drug needs to be stable in the blood circulation, disintegrate rapidly within the cells, and enable the efficient release of the toxin.

### Summary of the Invention

The inventors of the present application have creatively designed a novel linker structure, thereby providing the present invention.

### Linker-drug conjugate

The first aspect of the present invention relates to a linker-drug conjugate, or a pharmaceutically acceptable salt or solvate thereof, or a solvate of the salt, wherein, the linker-drug conjugate has a structure as shown in formula I:

M-X-D formula I

wherein,
M is a chemical structure comprising a maleimide (m) fragment or a fragment of cyclooctyne and its derivative (such as BCN, DIBO, DIBAC, and BARAC);
X is a linker fragment consisting of 1-5 amino acids or their derivatives, preferably selected from Ala-Ala-Ala-Ala-Asn, Ala-Ala-Ala-Asn, Ala-Ala-Asn, Ala-Asn, Asn, Ala-Ala-Ala-Ala-(3-cyano-alanine), Ala-Ala-Ala-(3-cyano-alanine), Ala-Ala-(3-cyano-alanine), Ala-(3-cyano-alanine), 3-cyano-alanine, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Ala-Gln, Ala-Ala-Ala-Gln, Ala-Ala-Gln, Ala-Gln, Ala-Ala-Ala-Ala-(2-amino-4-cyanobutanoic acid), Ala-Ala-Ala-(2-amino-4-cyanobutanoic acid), Ala-Ala-(2-amino-4-cyanobutanoic acid), Ala-(2-amino-4-cyanobutanoic acid), 2-amino-4-cyanobutanoic acid, Gly-Gly-Gly-Gln, Gly-Gly-Gln, Gly-Gln, Gln, and their deuterated compounds; and
D is a cytotoxin.

In the linker-drug conjugate of the present invention, the linker is composed of a chemical structure comprising a maleimide (m) fragment or a fragment of cyclooctyne and its derivative (such as BCN, DIBO, DIBAC, and BARAC) and a linker fragment consisting of 1-5 amino acids or their derivatives, and has the following characteristics: being stable in the blood circulation, being capable of disintegrating rapidly within the cell or in the tumor microenvironment, and enabling the efficient release of the toxin.

The chemical structure comprising a maleimide (m) fragment can be selected from 4-(N-maleimidomethyl)-cyclohexane-1-formyl (MCC), 6-maleimidocaproyl (MC), M-(PEG)ₙ, MC-(PEG)ₙ, MCC-(PEG)ₙ, etc., the structures of which are as shown below:

| | |
|---|---|
| MCC | 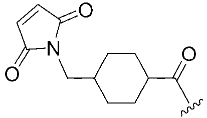 |
| MC | 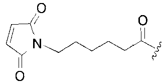 |
| M-(PEG)ₙ | 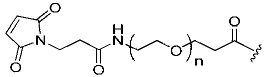 |
| MC-(PEG)ₙ | 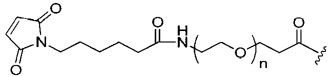 |
| MCC-(PEG)ₙ | 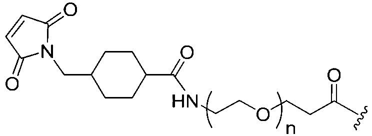 |

wherein, n represents the degree of polymerization of PEG, and is preferably an integer between 2 and 12.

In certain embodiments, the chemical structure comprising a maleimide (m) fragment is 6-maleimidocaproyl (MC). In certain embodiments, the chemical structure comprising a maleimide (m) fragment is MC-(PEG)ₙ, wherein, n is selected from 4, 8, and 12. The inventors have found that a lower degree of polymerization of PEG is, to a certain extent, beneficial for reducing the content of polymers (HMW%) in the ADC prepared from the linker-drug conjugate. Therefore, the degree of polymerization n of PEG is preferably 4.

The chemical structure comprising a cyclooctyne fragment can be selected from BCN, DIBO, DIBAC, BARAC, etc., the structures of which are as shown below:

| | |
|---|---|
| BCN | 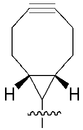 |
| DIBO | 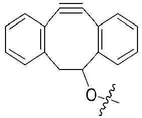 |
| DIBAC | 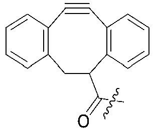 |
| BARAC | 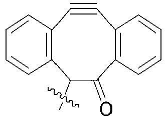 |

For most of the ADCs currently under research, the PAB structure (p-aminobenzyl-carbamoyl), as a self-releasing fragment in the linker, is an essential structure for the effective release of the Payload. However, the hydrophobicity of the PAB structure is likely to result in the generation of a relatively large amount of polymers in the preparation of ADCs. Therefore, further research and development of novel linker structures are still needed to overcome the above problem.

To this end, the inventors of the present application have found through experimental research that if D is a cytotoxin containing a hydroxyl (-OH), primary amine (-NH2), or secondary amine (-NHR) group, then the amino acid fragment comprised in the linker-drug conjugate of the present application is linked to the Payload containing a hydroxyl (-OH), primary amine (-NH₂), or secondary amine (-NHR) structure, enabling the release of the Payload by means of an enzymatic hydrolysis reaction in the case of removing the hydrophobic structure PAB, which can not only reduce the hydrophobicity of the Linker-Drug and reduce the content of the polymers produced in the preparation of the ADC, but also can exhibit the corresponding cell killing activity.

Therefore, in certain embodiments, D is a cytotoxin containing a hydroxyl (-OH), primary amine (-NH2), or secondary amine (-NHR) group.

In certain embodiments, the cytotoxin containing a hydroxyl (-OH), primary amine (-NH₂), or secondary amine (-NHR) group can be selected from topoisomerase I inhibitors such as camptothecin compounds and their derivatives (e.g., exatecan, 14-aminocamptothecin, 9-aminocamptothecin, and belotecan), topoisomerase II inhibitors such as doxorubicin compounds and their derivatives (e.g., Daunorubicin), maytansine compounds and their derivatives, calicheamicin compounds and their derivatives, DNA alkylating reagents such as duocarmycin compounds and their derivatives (e.g., Duocarmycin derivatives), and pyrrolobenzodiazepine compounds and their derivatives (e.g., PBD), tubulin inhibitors such as calendulin compounds and their derivatives (e.g., MMAE and its derivatives, or MMAF and its derivatives), Protac compounds and molecular glue compounds (e.g., lenalidomide), immunosuppressants such as rapamycin and its derivatives, and various other small-molecule compounds with cell killing activity. In certain embodiments, the cytotoxin containing a hydroxyl (-OH), primary amine (-NH₂), or secondary amine (-NHR) group is selected from Exatecan, belotecan, and Rapamycin. In certain embodiments, the cytotoxin containing a hydroxyl (-OH) group is Rapamycin. In certain embodiments, the cytotoxin containing a secondary amine (-NHR) group is belotecan. In certain embodiments, the cytotoxin containing a primary amine (-NH₂) group is Exatecan.

In certain embodiments, the linker fragment consisting of 1-5 amino acids or their derivatives in formula I is selected from Ala-Ala-Ala-Ala-Asn, Ala-Ala-Ala-Asn, Ala-Ala-Asn, Ala-Asn, Asn, Ala-Ala-Ala-Ala-(3-cyano-alanine), Ala-Ala-Ala-(3-cyano-alanine), Ala-Ala-(3-cyano-alanine), Ala-(3-cyano-alanine), 3-cyano-alanine, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Ala-Gln, Ala-Ala-Ala-Gln, Ala-Ala-Gln, Ala-Gln, Ala-Ala-Ala-Ala-(2-amino-4-cyanobutanoic acid), Ala-Ala-Ala-(2-amino-4-cyanobutanoic acid), Ala-Ala-(2-amino-4-cyanobutanoic acid), Ala-(2-amino-4-cyanobutanoic acid), 2-amino-4-cyanobutanoic acid, Gly-Gly-Gly-Gln, Gly-Gly-Gln, Gly-Gln, Gln, and their deuterated compounds.

The amino acid monomer or its derivative, or the fragment consisting of multiple amino acids or their derivatives in formula I can be recognized by an enzyme in the lysosomes of tumor cells. The enzyme can dissociate the ester bond or amide bond (peptide bond) formed between the carboxyl group in this type of compound and the compound containing a hydroxyl (-OH), primary amine (-NH₂), or secondary amine (-NHR), without the need for an additional self-immolative moiety.

In certain embodiments, the linker fragment consisting of 1-5 amino acids or their derivatives in formula I is selected from Ala-Ala-Ala-Ala-Asn, Ala-Ala-Ala-Asn, Ala-Ala-Asn, Ala-Asn, Asn, Ala-Ala-Ala-Ala-(3-cyano-L-alanine), Ala-Ala-Ala-(3-cyano-L-alanine), Ala-Ala-(3-cyano-L-alanine), Ala-(3-cyano-L-alanine), 3-cyano-L-alanine, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Ala-Gln, Ala-Ala-Ala-Gln, Ala-Ala-Gln, Ala-Gln, Ala-Ala-Ala-Ala-((*S*)-2-amino-4-cyanobutanoic acid), Ala-Ala-Ala-((*S*)-2-amino-4-cyanobutanoic acid), Ala-Ala-((*S*)-2-amino-4-cyanobutanoic acid), Ala-((*S*)-2-amino-4-cyanobutanoic acid), (*S*)-2-amino-4-cyanobutanoic acid, Gly-Gly-Gly-Gln, Gly-Gly-Gln, Gly-Gln, and Gln.

In certain embodiments, the linker fragment is selected from linker fragments containing asparagine (Asn), e.g., Ala-Ala-Ala-Ala-Asn, Ala-Ala-Ala-Asn, Ala-Ala-Asn, Ala-Asn, Asn, Pro-Asn, and Lys-Asn. In certain embodiments, the linker fragment is Ala-Ala-Asn (AAN).

In certain embodiments, the linker-drug conjugate of the present invention is MCC-AAQ-Exatecan which has the structure as shown below:

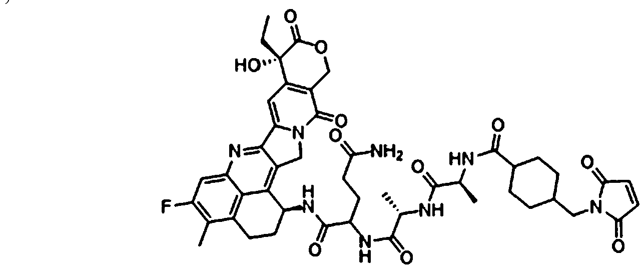

### Antibody-drug conjugate

The second aspect of the present invention relates to an antibody-drug conjugate, or a pharmaceutically acceptable salt or solvate thereof, or a solvate of the salt, wherein, the antibody-drug conjugate is formed by conjugating the linker-drug conjugate described in the first aspect of the present invention to an antibody.

In certain embodiments, the antibody-drug conjugate has a structure as shown in formula II:

Ab-(L-D)ₚ formula II

wherein,
Ab is an antibody;
L is a linker, with a structure of M'-X, wherein, M' is linked to Ab, and X is linked to D;
M' is a chemical structure formed by linking M as defined above to Ab through a maleimide group;
X is a linker fragment consisting of 1-5 amino acids or their derivatives as defined above;
D is a cytotoxin as defined above; and
p is any numerical value between 2 and 8 (e.g., 2, 2.5, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, or 8, or e.g., 2-2.5, 2.5-3, 3-3.5, 3.5-4, 4-4.5, 4.5-5, 5-5.5, 5.5-6, 6-6.5, 6.5-7, 7-7.5, or 7.5-8).

In formula II, L-D means that the linker is linked to the cytotoxin via a covalent bond to form an L-D molecule; and Ab-(L-D)ₚ means that p L-D molecules are conjugated to Ab via covalent bonds.

In the present invention, the drug-to-antibody ratio (DAR) refers to the number of drug molecules conjugated to the antibody (e.g., p in formula II). The number of drug molecules contained in the antibody-drug conjugate described herein can be either an integer or a decimal. The number, whether an integer or a decimal, refers to the average number of drug molecules conjugated to each antibody. "p is any numerical value between 2 and 8" means that p may be either any integer between 2 and 8 (including endpoints 2 and 8) or any decimal between 2 and 8, e.g., 2.3, 3.9, 4.0, or 4.2. Moreover, those skilled in the art can understand that even if the same preparation method is used, the DAR values of antibody-drug conjugates prepared in different batches are not necessarily the same, for example, they can fluctuate within a range of no more than 0.5 up and down.

The drug-to-antibody ratio (DAR) can be verified by conventional analytic means, such as mass spectrometry, ELISA assay, HIC, and HPLC. ADCs may also be measured for quantitative distribution in terms of p. In some cases, the separation of homogeneous ADCs with p as a certain numerical value from ADCs with other drug loads, followed by purification and verification can be achieved by means such as reversed-phase HPLC or electrophoresis.

In the present invention, when the linker-drug conjugate is conjugated to the antibody Ab via a covalent bond, one way is to conjugating to the sulfhydryl group in the antibody through maleimide to form an -S-succinimide structure. For example, when MCC-AAQ-Exatecan is conjugated to Ab via a covalent bond, the structural formula of the formed ADC is as shown below:

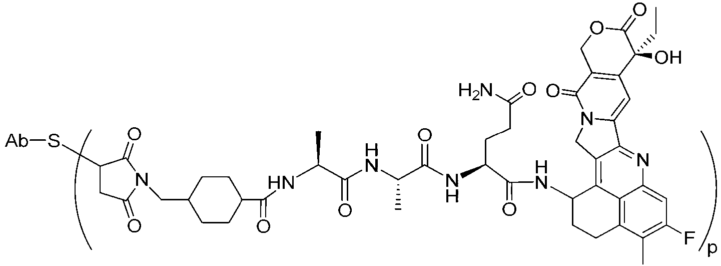

In the ADC with a maleimide linker formed as described above, -S- can either be an externally introduced sulfhydryl group through engineering or a sulfhydryl group contained within the antibody itself after the antibody Ab is reduced and thus its disulfide bonds are broken.

Another way is to conjugating cyclooctyne to an azide group (-N₃) introduced into the modified antibody to form a triazole structure. For example, when BCN-AAQ-Exatecan is conjugated to Ab via a covalent bond, the structural formula of the formed ADC is as shown below:

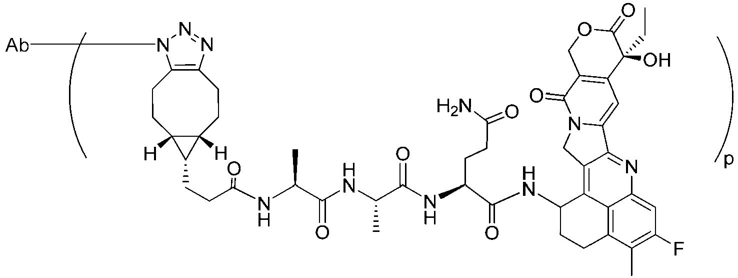

There is no particular limitation on the antibody that can be used in the present invention, and it can be selected from murine antibodies, rabbit antibodies, phage display-derived antibodies, yeast display-derived antibodies, chimeric antibodies, humanized antibodies, fully human antibodies, antibody fragments, bispecific antibodies, and multispecific antibodies.

In certain embodiments, the antibody is a monoclonal antibody, which is selected non-restrictively from: HER2 antibodies and EGFR antibodies.

In certain embodiments, the antibody is a HER2 antibody. In certain embodiments, the antibody consists of a light chain and a heavy chain, wherein, the light chain comprises CDR-L1, CDR-L2, and CDR-L3 with the amino acid sequences as set forth in SEQ ID NO: 1, SEQ ID NO: 2, and SEQ ID NO: 3, respectively. In certain embodiments, the heavy chain comprises CDR-H1, CDR-H2, and CDR-H3 with the amino acid sequences as set forth in SEQ ID NO: 4, SEQ ID NO: 5, and SEQ ID NO: 6, respectively.

In certain embodiments, the light chain comprises a light chain variable region with the amino acid sequence of SEQ ID NO: 7. In certain embodiments, the light chain further comprises a light chain constant region with the amino acid sequence of SEQ ID NO: 8. In certain embodiments, the amino acid sequence of the light chain is SEQ ID NO: 9.

In certain embodiments, the heavy chain comprises a heavy chain variable region with the amino acid sequence of SEQ ID NO: 10. In certain embodiments, the heavy chain further comprises a heavy chain constant region with the amino acid sequence of SEQ ID NO: 11. In certain embodiments, the amino acid sequence of the heavy chain is SEQ ID NO: 12.

In certain embodiments, the antibody is an EGFR antibody. In certain embodiments, the antibody consists of a light chain and a heavy chain, wherein, the light chain comprises CDR-L1, CDR-L2, and CDR-L3 with the amino acid sequences as set forth in SEQ ID NO: 13, SEQ ID NO: 14, and SEQ ID NO: 15, respectively. In certain embodiments, the heavy chain comprises CDR-H1, CDR-H2, and CDR-H3 with the amino acid sequences as set forth in SEQ ID NO: 16, SEQ ID NO: 17, and SEQ ID NO: 18, respectively.

In certain embodiments, the light chain comprises a light chain variable region with the amino acid sequence of SEQ ID NO: 19. In certain embodiments, the light chain further comprises a light chain constant region with the amino acid sequence of SEQ ID NO: 20. In certain embodiments, the amino acid sequence of the light chain is SEQ ID NO: 21.

In certain embodiments, the heavy chain comprises a heavy chain variable region with the amino acid sequence of SEQ ID NO: 22. In certain embodiments, the heavy chain further comprises a heavy chain constant region with the amino acid sequence of SEQ ID NO: 23. In certain embodiments, the amino acid sequence of the heavy chain is SEQ ID NO: 24.

The information of part of the sequences involved in the present invention is provided in Table 1.

**Table 1: Description of sequences**

| SEQ ID NO: | Description | Sequence |
|---|---|---|
| HER2 antibody sequences | | |
| 1 | Amino acid sequence of CDR-L1 of the HER2 antibody | RASQDVNTAVA |
| 2 | Amino acid sequence of CDR-L2 of the HER2 antibody | SASFLYS |
| 3 | Amino acid sequence of CDR-L3 of the HER2 antibody | QQHYTTPPT |
| 4 | Amino acid sequence of CDR-H1 of the HER2 antibody | GFNIKDTYIH |
| 5 | Amino acid sequence of CDR-H2 of the HER2 antibody | RIYPTNGYTRYADSVKG |
| 6 | Amino acid sequence of CDR-H3 of the HER2 antibody | WGGDGFYAMDY |
| 7 | Amino acid sequence of the light chain variable region of the HER2 antibody | DIQMTQSPSSLSASVGDRVTITCRASQDVNTA VAWYQQKPGKAPKLLIYSASFLYSGVPSRFSG SRSGTDFTLTISSLQPEDFATYYCQQHYTTPPT F |
| 8 | Amino acid sequence of the light chain constant region of the HER2 antibody | GQGTKVEIKRTVAAPSVFIFPPSDEQLKSGTAS VVCLLNNFYPREAKVQWKVDNALQSGNSQES VTEQDSKDSTYSLSSTLTLSKADYEKHKVYAC EVTHQGLSSPVTKSFNRGEC |
| 9 | Amino acid sequence of the light chain of the HER2 antibody | DIQMTQSPSSLSASVGDRVTITCRASQDVNTA VAWYQQKPGKAPKLLIYSASFLYSGVPSRFSG SRSGTDFTLTISSLQPEDFATYYCQQHYTTPPT FGQGTKVEIKRTVAAPSVFIFPPSDEQLKSGTA SVVCLLNNFYPREAKVQWKVDNALQSGNSQE SVTEQDSKDSTYSLSSTLTLSKADYEKHKVYA CEVTHQGLSSPVTKSFNRGEC |
| 10 | Amino acid sequence of the heavy chain variable region of the HER2 antibody | EVQLVESGGGLVQPGGSLRLSCAASGFNIKDT YIHWVRQAPGKGLEWVARIYPTNGYTRYADS VKGRFTISADTSKNTAYLQMNSLRAEDTAVY YCSRWGGDGFYAMDYWGQGTLVTVSSASTK G |
| 11 | Amino acid sequence of the heavy chain constant region of the HER2 antibody | PSVFPLAPSSKSTSGGTAALGCLVKDYFPEPVT VSWNSGALTSGVHTFPAVLQSSGLYSLSSVVT VPSSSLGTQTYICNVNHKPSNTKVDKKVEPKS CDKTHTCPPCPAPELLGGPSVFLFPPKPKDTLM ISRTPEVTCVVVDVSHEDPEVKFNWYVDGVE VHNAKTKPREEQYNSTYRVVSVLTVLHQDWL NGKEYKCKVSNKALPAPIEKTISKAKGQPREP QVYTLPPSREEMTKNQVSLTCLVKGFYPSDIA VEWESNGQPENNYKTTPPVLDSDGSFFLYSKL TVDKSRWQQGNVFSCSVMHEALHNHYTQKS LSLSPGK |
| 12 | Amino acid sequence of the heavy chain of the HER2 antibody | EVQLVESGGGLVQPGGSLRLSCAASGFNIKDT YIHWVRQAPGKGLEWVARIYPTNGYTRYADS VKGRFTISADTSKNTAYLQMNSLRAEDTAVY YCSRWGGDGFYAMDYWGQGTLVTVSSASTK GPSVFPLAPSSKSTSGGTAALGCLVKDYFPEPV TVSWNSGALTSGVHTFPAVLQSSGLYSLSSVV TVPSSSLGTQTYICNVNHKPSNTKVDKKVEPK SCDKTHTCPPCPAPELLGGPSVFLFPPKPKDTL MISRTPEVTCVVVDVSHEDPEVKFNWYVDGV EVHNAKTKPREEQYNSTYRVVSVLTVLHQDW LNGKEYKCKVSNKALPAPIEKTISKAKGQPRE PQVYTLPPSREEMTKNQVSLTCLVKGFYPSDI AVEWESNGQPENNYKTTPPVLDSDGSFFLYSK LTVDKSRWQQGNVFSCSVMHEALHNHYTQK SLSLSPGK |

| EGFR antibody sequences | | |
|---|---|---|
| 13 | Amino acid sequence of CDR-L1 of the EGFR antibody | QSIGTN |
| 14 | Amino acid sequence of CDR-L2 of the EGFR antibody | YAS |
| 15 | Amino acid sequence of CDR-L3 of the EGFR antibody | QQNNEWPTS |
| 16 | Amino acid sequence of CDR-H1 of the EGFR antibody | GFSLSNYD |
| 17 | Amino acid sequence of CDR-H2 of the EGFR antibody | IWSGGNT |
| 18 | Amino acid sequence of CDR-H3 of the EGFR antibody | ARALDYYDYEFAY |
| 19 | Amino acid sequence of the light chain variable region of the EGFR antibody | EIVLTQSPDFQSVTPKEKVTITCRASQSIGTNIH WYQQKPDQSPKLLIKYASESISGIPSRFSGSGSG TD |
| 20 | Amino acid sequence of the light chain constant region of the EGFR antibody | GQGTKLEIKRTVAAPSVFIFPPSDEQLKSGTAS VVCLLNNFYPREAKVQWKVDNALQSGNSQES VTEQDSKDSTYSLSSTLTLSKADYEKHKVYAC EVTHQGLSSPVTKSFNRGEC |
| 21 | Amino acid sequence of the light chain of the EGFR antibody | EIVLTQSPDFQSVTPKEKVTITCRASQSIGTNIH WYQQKPDQSPKLLIKYASESISGIPSRFSGSGSG TDFTLTINSLEAEDAATYYCQQNNEWPTSFGQ GTKLEIKRTVAAPSVFIFPPSDEQLKSGTASVV CLLNNFYPREAKVQWKVDNALQSGNSQESVT EQDSKDSTYSLSSTLTLSKADYEKHKVYACEV THQGLSSPVTKSFNRGEC |
| 22 | Amino acid sequence of the heavy chain variable region of the EGFR antibody | QVQLQESGPGLVKPSETLSLTCTVSGFSLSNYD VHWVRQAPGKGLEWLGVIWSGGNTDYNTPF TSRLTISVDTSKNQFSLKLSSVTAADTAVYYC ARALDYYDYEFAYW |
| 23 | Amino acid sequence of the heavy chain constant region of the EGFR antibody | GQGTLVTVSSASTKGPSVFPLAPSSKSTSGGTA ALGCLVKDYFPEPVTVSWNSGALTSGVHTFPA VLQSSGLYSLSSVVTVPSSSLGTQTYICNVNHK PSNTKVDKRVEPKSCDKTHTCPPCPAPELLGG PSVFLFPPKPKDTLMISRTPEVTCVVVDVSHED PEVKFNWYVDGVEVHNAKTKPREEQYNSTYR VVSVLTVLHQDWLNGKEYKCKVSNKALPAPI EKTISKAKGQPREPQVYTLPPSREEMTKNQVS LTCLVKGFYPSDIAVEWESNGQPENNYKTTPP VLDSDGSFFLYSKLTVDKSRWQQGNVFSCSV MHEALHNHYTQKSLSLSPGK |
| 24 | Amino acid sequence of the heavy chain of the EGFR antibody | QVQLQESGPGLVKPSETLSLTCTVSGFSLSNYD VHWVRQAPGKGLEWLGVIWSGGNTDYNTPF TSRLTISVDTSKNQFSLKLSSVTAADTAVYYC ARALDYYDYEFAYWGQGTLVTVSSASTKGPS VFPLAPSSKSTSGGTAALGCLVKDYFPEPVTVS WNSGALTSGVHTFPAVLQSSGLYSLSSVVTVP SSSLGTQTYICNVNHKPSNTKVDKRVEPKSCD KTHTCPPCPAPELLGGPSVFLFPPKPKDTLMIS RTPEVTCVVVDVSHEDPEVKFNWYVDGVEVH NAKTKPREEQYNSTYRVVSVLTVLHQDWLNG KEYKCKVSNKALPAPIEKTISKAKGQPREPQV YTLPPSREEMTKNQVSLTCLVKGFYPSDIAVE |
| | | WESNGQPENNYKTTPPVLDSDGSFFLYSKLTV DKSRWQQGNVFSCSVMHEALHNHYTQKSLSL SPGK |

### Pharmaceutical composition and pharmaceutical use

The third aspect of the present invention provides a pharmaceutical composition comprising the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt.

In some embodiments, the pharmaceutical composition further comprises at least one of a chemotherapeutic drug, an immunotherapeutic drug, and an immunosuppressant for treating a tumor.

In some embodiments, the chemotherapeutic drug is, for example, doxorubicin (Adriamycin), cyclophosphamide, a taxane (e.g., paclitaxel (Taxol) or docetaxel (Taxotere)), capecitabine (Xeloda), gemcitabine (Gemzar), vinorelbine (Navelbine), tamoxifen, an aromatase inhibitor (Arimidex, Femara, or Aromasin), 5-FU + folinic acid, irinotecan (Camptosar), oxaliplatin, cisplatin, carboplatin, estramustine, mitoxantrone (Novantrone), prednisone, vincristine (Oncovin), adriamycin, prednisone, etc., or a combination thereof.

In some embodiments, the immunotherapeutic drug is, for example, a PD-1 antibody, a PD-L1 antibody, an EGFR monoclonal antibody, a CD20 monoclonal antibody, a HER2 monoclonal antibody (e.g., Trastuzumab, Trastuzumab biosimilar, and Trastuzumab-dkst), etc., or a combination thereof.

In some embodiments, the immunosuppressant is selected from: (1) glucocorticoids, such as cortisone and prednisone; (2) microbial metabolites, such as cyclosporine and Fujimycin; (3) antimetabolites, such as azathioprine and 6-mercaptopurine; (4) polyclonal and monoclonal anti-lymphocyte antibodies, such as anti-lymphocyte globulin and OKT3; and (5) alkylating agents, such as cyclophosphamide. In some specific embodiments, the immunosuppressant is, for example, methylprednisolone, prednisone, azathioprine, Prograf, Zenapax, Simulect, cyclosporine, tacrolimus, rapamycin, mycophenolate mofetil, mizoribine, cyclophosphamide, or fingolimod.

In some embodiments, the pharmaceutical composition further comprises at least one pharmaceutical auxiliary material.

The fourth aspect of the present invention provides use of the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt in the preparation of a medicament for the prevention and/or treatment of a tumor.

The fifth aspect of the present invention provides a method for treating and/or preventing a tumor, comprising: administering to a subject in need thereof a therapeutically and/or prophylactically effective amount of the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt.

The "tumor" as described in the present invention refers to a lesion formed by the excessive proliferation of local cells in an organ or tissue, including hematological tumors and solid tumors (including benign tumors and malignant tumors). In certain embodiments, the tumor is selected from tumors that are positive for or highly express the following markers: HER2 and EGFR.

In certain embodiments, the tumor is selected from: breast cancer, melanoma, meningioma, soft tissue sarcoma, salivary gland tumor, primary liver cancer, intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia, gastric cancer, nasopharyngeal cancer, laryngeal cancer, oral cancer, esophageal cancer, lung cancer (e.g., non-small cell lung cancer and small cell lung cancer), kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi sarcoma, non-melanoma skin cancer (including squamous cell carcinoma and basal cell carcinoma), hemangioma, glioma, and brain glioma.

### Preparation method

The sixth aspect of the present invention provides a method for preparing the aforementioned linker-drug conjugate, or the pharmaceutically acceptable salt or solvate thereof, or the solvate of the salt, comprising the following steps:
1. preparing a linker fragment X consisting of 1-5 amino acids or their derivatives;
2. linking X to a chemical structure M comprising a maleimide (m) fragment or a cyclooctyne fragment to obtain M-X; and
3. linking a cytotoxin D to M-X to obtain the linker-drug conjugate M-X-D.

The above preparation method will be further illustrated by taking MCC-AAQ-Exatecan as an example below. The exemplary preparation process of MCC-AAQ-Exatecan is as follows:
1. reacting Fmoc-Gln with Exatecan to obtain Intermediate 1, wherein, the structural formula of Intermediate 1 is as shown below: 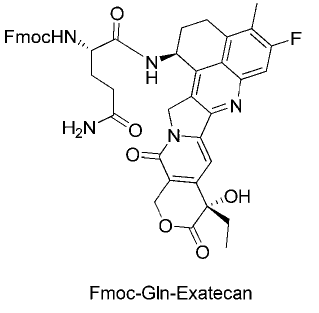
2. removing the Fmoc protecting group from Intermediate 1 using DEA to obtain Intermediate 2, wherein, the structural formula of Intermediate 2 is as shown below: 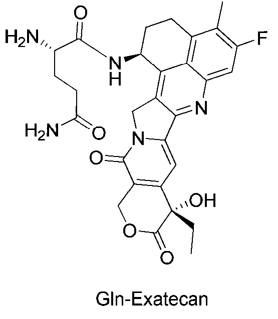
3. reacting Intermediate 2 with Fmoc-Ala-Ala to obtain Intermediate 3, wherein, the structural formula of Intermediate 3 is as shown below: 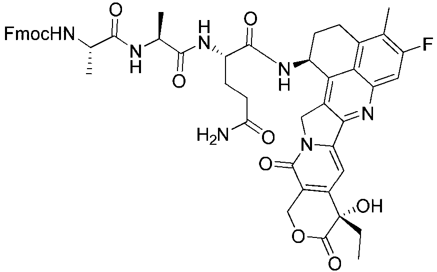 Fmoc-Ala-Ala-Gln-Exatecan
4. removing the Fmoc protecting group from Intermediate 3 to obtain Intermediate 4, wherein, the structural formula of Intermediate 4 is as shown below: 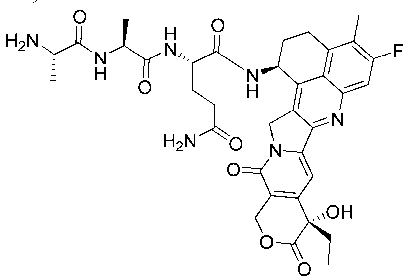 Ala-Ala-Gln-Exatecan
5. and condensing Intermediate 4 with MCC to obtain the product, wherein, the structural formula of the product is as shown below:

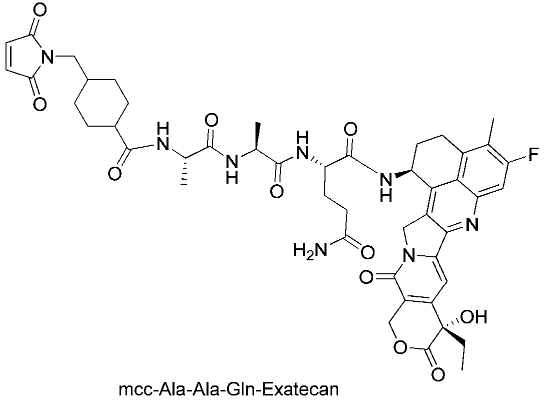

The seventh aspect of the present invention provides a method for preparing the aforementioned antibody-drug conjugate, or the pharmaceutically acceptable salt or solvate thereof, or the solvate of the salt, comprising the following steps:
1. taking a certain weight of a monoclonal antibody, diluting with PBS buffer, and adjusting the pH of the antibody to around 7.5; detecting the protein concentration, weighing the net weight of the antibody solution, and calculating the total protein amount; and adding a TCEP solution to the antibody, and reacting at room temperature while continuously shaking;
2. adding an excess of a cytotoxic drug solution to the reduced antibody solution, mixing evenly, and then reacting at room temperature while continuously mixing; and after the reaction is completed, adding an excess of N-acetylcysteine solution to the reaction mixture, and reacting at room temperature while continuously mixing; and
3. purifying the conjugated product using an ultrafiltration centrifuge tube, exchanging into a storage solution, and then filtering through a sterilizing filter to obtain the antibody-drug conjugate.

In addition, the present invention also provides the following technical solutions:

### Linker-drug conjugate

The eighth aspect of the present invention relates to a linker-drug conjugate, or a pharmaceutically acceptable salt or solvate thereof, or a solvate of the salt, wherein, the linker-drug conjugate has a structure as shown in formula I:

M-X-D formula I

wherein,
M is a chemical structure comprising a maleimide (m) fragment or a fragment of cyclooctyne and its derivative (such as BCN, DIBO, DIBAC, and BARAC);
X is a linker fragment consisting of 1-4 amino acids, selected from Ala-Ala-Ala-Asn, Ala-Ala-Asn, Ala-Asn, Asn, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Gln, Ala-Ala-Gln, Ala-Gln, Gly-Gly-Gly-Gln, Gly-Gly-Gln, Gly-Gln, and Gln; and
D is a cytotoxin containing a primary amine (-NH₂) group.

In the linker-drug conjugate of the present invention, the linker is composed of a chemical structure comprising a maleimide (m) fragment or a fragment of cyclooctyne and its derivative (such as BCN, DIBO, DIBAC, and BARAC) and a linker fragment consisting of 1-4 amino acids, and has the following characteristics: being stable in the blood circulation, being capable of disintegrating rapidly within the cell or in the tumor microenvironment, and enabling the efficient release of the toxin.

The chemical structure comprising a maleimide (m) fragment can be selected from 4-(N-maleimidomethyl)-cyclohexane-1-formyl (mcc), 6-maleimidocaproyl (mc), m-(PEG)ₙ, mc-(PEG)ₙ, mcc-(PEG)ₙ, etc., the structures of which are as shown below:

| | |
|---|---|
| mcc | 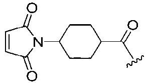 |
| mc | 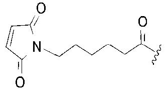 |
| m-(PEG)ₙ | 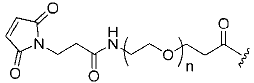 |
| mc-(PEG)ₙ | 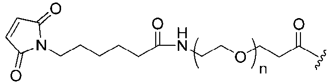 |
| mcc-(PEG)ₙ | 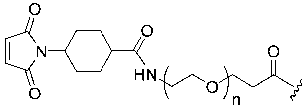 |

| | |
|---|---|
| wherein, n represents the degree of polymerization of PEG, and is preferably an integer between 2 and 12. | |

In certain embodiments, the chemical structure comprising a maleimide (m) fragment is 6-maleimidocaproyl (mc). In certain embodiments, the chemical structure comprising a maleimide (m) fragment is mc-(PEG)ₙ, wherein, n is selected from 4, 8, and 12. The inventors have found that a lower degree of polymerization of PEG is, to a certain extent, beneficial for reducing the content of polymers (HMW%) in the ADC prepared from the linker-drug conjugate. Therefore, the degree of polymerization n of PEG is preferably 4.

The chemical structure comprising a cyclooctyne fragment can be selected from BCN, DIBO, DIBAC, BARAC, etc., the structures of which are as shown below:

| | |
|---|---|
| BCN | 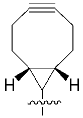 |
| DIBO | 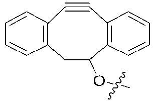 |
| DIBAC | 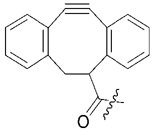 |
| BARAC | 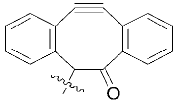 |

In formula I, the linker fragment consisting of 1-4 amino acids is selected from Ala-Ala-Ala-Asn, Ala-Ala-Asn, Ala-Asn, Asn, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Gln, Ala-Ala-Gln, Ala-Gln, Gly-Gly-Gly-Gln, Gly-Gly-Gln, Gly-Gln, and Gln. The above amino acid monomer or the fragment consisting of multiple amino acids can be recognized by a specific enzyme in the lysosomes of tumor cells. The enzyme can dissociate the amide bond (peptide bond) formed between the carboxyl group in this type of compound and the compound containing a primary amine, without the need for an additional self-immolative moiety.

In certain embodiments, the linker fragment is selected from linker fragments containing asparagine (Asn), e.g., Ala-Ala-Ala-Asn, Ala-Ala-Asn, Ala-Asn, Asn, Pro-Asn, and Lys-Asn. Asparagine (Asn) can be recognized by Legumain. In addition to being present in the lysosomes of tumor cells, Legumain is abundantly distributed outside tumor cells, thus enabling the release of small molecules extracellularly, which endows them with the by-stander effect and a good killing effect on cells with low endocytic target sites. In certain embodiments, the linker fragment is Ala-Ala-Asn (AAN).

The cytotoxin containing a primary amine (-NH₂) group can be selected from topoisomerase I inhibitors such as camptothecin compounds and their derivatives (e.g., exatecan, 14-aminocamptothecin, and 9-aminocamptothecin), topoisomerase II inhibitors such as doxorubicin compounds and their derivatives (e.g., Daunorubicin), maytansine compounds and their derivatives, calicheamicin compounds and their derivatives, DNA alkylating reagents such as duocarmycin compounds and their derivatives (e.g., Duocarmycin derivatives), and pyrrolobenzodiazepine compounds and their derivatives (e.g., PBD), tubulin inhibitors such as calendulin compounds and their derivatives (e.g., desmethyl MMAE or desmethyl MMAF), Protac compounds and molecular glue compounds (e.g., lenalidomide), and various other small-molecule compounds with cell killing activity. In certain embodiments, the cytotoxin containing a primary amine (-NH₂) group is Exatecan.

In certain embodiments, the cytotoxin containing a primary amine (-NH₂) group is selected from Exatecan, 14-aminocamptothecin (14-AC), 9-aminocamptothecin (9-AC), desmethyl MMAE, desmethyl MMAF, Daunorubicin, and lenalidomide.

In certain embodiments, the linker-drug conjugate of the present invention is mc-AAN-Exatecan, which has the structure as shown below:

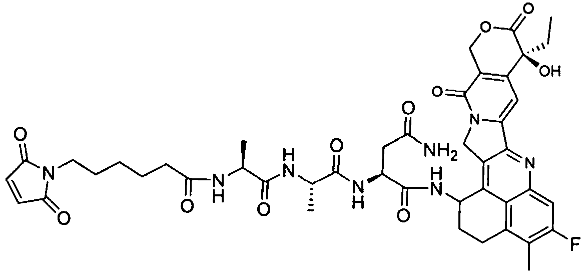

### Antibody-drug conjugate

The ninth aspect of the present invention relates to an antibody-drug conjugate, or a pharmaceutically acceptable salt or solvate thereof, or a solvate of the salt, wherein, the antibody-drug conjugate is formed by conjugating the linker-drug conjugate described in the eighth aspect of the present invention to an antibody.

In certain embodiments, the antibody-drug conjugate has a structure as shown in formula II:

Ab-(L-D)ₚ formula II

wherein,
Ab is an antibody;
L is a linker, with a structure of M'-X, wherein, M' is linked to Ab, and X is linked to D;
M' is a chemical structure formed by linking M as defined above to Ab through a maleimide group;
X is a linker fragment consisting of 1-4 amino acids as defined above;
D is a cytotoxin containing a primary amine (-NH₂) group as defined above; and
p is any numerical value between 2 and 8 (e.g., 2, 2.5, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, or 8, or e.g., 2-2.5, 2.5-3, 3-3.5, 3.5-4, 4-4.5, 4.5-5, 5-5.5, 5.5-6, 6-6.5, 6.5-7, 7-7.5, or 7.5-8).

In formula II, L-D means that the linker is linked to the cytotoxin via a covalent bond to form an L-D molecule; and Ab-(L-D)ₚ means that p L-D molecules are conjugated to Ab via covalent bonds.

In the present invention, the drug-to-antibody ratio (DAR) refers to the number of drug molecules conjugated to the antibody (e.g., p in formula I). The number of drug molecules contained in the antibody-drug conjugate described herein can be either an integer or a decimal. The number, whether an integer or a decimal, refers to the average number of drug molecules conjugated to each antibody. "p is any numerical value between 2 and 8" means that p may be either any integer between 2 and 8 (including endpoints 2 and 8) or any decimal between 2 and 8, e.g., 2.3, 3.9, 4.0, or 4.2. Moreover, those skilled in the art can understand that even if the same preparation method is used, the DAR values of antibody-drug conjugates prepared in different batches are not necessarily the same, for example, they can fluctuate within a range of no more than 0.5 up and down.

The drug-to-antibody ratio (DAR) can be verified by conventional analytic means, such as mass spectrometry, ELISA assay, HIC, and HPLC. ADCs may also be measured for quantitative distribution in terms of p. In some cases, the separation of homogeneous ADCs with p as a certain numerical value from ADCs with other drug loads, followed by purification and verification can be achieved by means such as reversed-phase HPLC or electrophoresis.

In the present invention, when the linker-drug conjugate is conjugated to the antibody Ab via a covalent bond, one way is to conjugating to the sulfhydryl group in the antibody through maleimide to form an -S-succinimide structure. For example, when mc-AAN-Exatecan is conjugated to Ab via a covalent bond, the structural formula of the formed ADC is as shown below:

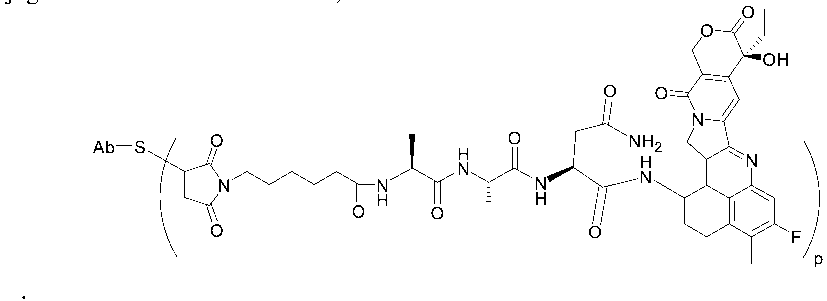

In the ADC with a maleimide linker formed as described above, -S- can either be an externally introduced sulfhydryl group through engineering or a sulfhydryl group contained within the antibody itself after the antibody Ab is reduced and thus its disulfide bonds are broken.

Another way is to linking the chemical structure of the fragment of cyclooctyne and its derivative (such as BCN, DIBO, DIBAC, and BARAC) to the antibody, wherein, the cyclooctyne is conjugated to an azide group (-N₃) introduced into the modified antibody to form a triazole structure. For example, when BCN-AAN-Exatecan is conjugated to Ab via a covalent bond, the structural formula of the formed ADC is as shown below:

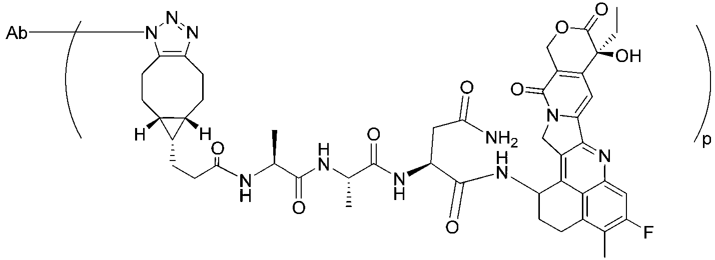

There is no particular limitation on the antibody that can be used in the present invention, and it can be selected from murine antibodies, rabbit antibodies, phage display-derived antibodies, yeast display-derived antibodies, chimeric antibodies, humanized antibodies, fully human antibodies, antibody fragments, bispecific antibodies, and multispecific antibodies.

In certain embodiments, the antibody is a monoclonal antibody, which is selected non-restrictively from: HER2 antibodies and EGFR antibodies.

In certain embodiments, the antibody is a HER2 antibody. In certain embodiments, the antibody consists of a light chain and a heavy chain, wherein, the light chain comprises CDR-L1, CDR-L2, and CDR-L3 with the amino acid sequences as set forth in SEQ ID NO: 1, SEQ ID NO: 2, and SEQ ID NO: 3, respectively. In certain embodiments, the heavy chain comprises CDR-H1, CDR-H2, and CDR-H3 with the amino acid sequences as set forth in SEQ ID NO: 4, SEQ ID NO: 5, and SEQ ID NO: 6, respectively.

In certain embodiments, the light chain comprises a light chain variable region with the amino acid sequence of SEQ ID NO: 7. In certain embodiments, the light chain further comprises a light chain constant region with the amino acid sequence of SEQ ID NO: 8. In certain embodiments, the amino acid sequence of the light chain is SEQ ID NO: 9.

In certain embodiments, the heavy chain comprises a heavy chain variable region with the amino acid sequence of SEQ ID NO: 10. In certain embodiments, the heavy chain further comprises a heavy chain constant region with the amino acid sequence of SEQ ID NO: 11. In certain embodiments, the amino acid sequence of the heavy chain is SEQ ID NO: 12.

In certain embodiments, the antibody is an EGFR antibody. In certain embodiments, the antibody consists of a light chain and a heavy chain, wherein, the light chain comprises CDR-L1, CDR-L2, and CDR-L3 with the amino acid sequences as set forth in SEQ ID NO: 13, SEQ ID NO: 14, and SEQ ID NO: 15, respectively. In certain embodiments, the heavy chain comprises CDR-H1, CDR-H2, and CDR-H3 with the amino acid sequences as set forth in SEQ ID NO: 16, SEQ ID NO: 17, and SEQ ID NO: 18, respectively.

In certain embodiments, the light chain comprises a light chain variable region with the amino acid sequence of SEQ ID NO: 19. In certain embodiments, the light chain further comprises a light chain constant region with the amino acid sequence of SEQ ID NO: 20. In certain embodiments, the amino acid sequence of the light chain is SEQ ID NO: 21.

In certain embodiments, the heavy chain comprises a heavy chain variable region with the amino acid sequence of SEQ ID NO: 22. In certain embodiments, the heavy chain further comprises a heavy chain constant region with the amino acid sequence of SEQ ID NO: 23. In certain embodiments, the amino acid sequence of the heavy chain is SEQ ID NO: 24.

The information of part of the sequences involved in the present invention is provided in Table 1 above.

### Pharmaceutical composition and pharmaceutical use

The tenth aspect of the present invention provides a pharmaceutical composition comprising the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt.

In some embodiments, the pharmaceutical composition further comprises at least one of a chemotherapeutic drug, an immunotherapeutic drug, and an immunosuppressant for treating a tumor.

In some embodiments, the chemotherapeutic drug is, for example, doxorubicin (Adriamycin), cyclophosphamide, a taxane (e.g., paclitaxel (Taxol) or docetaxel (Taxotere)), capecitabine (Xeloda), gemcitabine (Gemzar), vinorelbine (Navelbine), tamoxifen, an aromatase inhibitor (Arimidex, Femara, or Aromasin), 5-FU + folinic acid, irinotecan (Camptosar), oxaliplatin, cisplatin, carboplatin, estramustine, mitoxantrone (Novantrone), prednisone, vincristine (Oncovin), adriamycin, prednisone, etc., or a combination thereof.

In some embodiments, the immunotherapeutic drug is, for example, a PD-1 antibody, a PD-L1 antibody, an EGFR monoclonal antibody, a CD20 monoclonal antibody, a HER2 monoclonal antibody (e.g., Trastuzumab, Trastuzumab biosimilar, and Trastuzumab-dkst), etc., or a combination thereof.

In some embodiments, the immunosuppressant is selected from: (1) glucocorticoids, such as cortisone and prednisone; (2) microbial metabolites, such as cyclosporine and Fujimycin; (3) antimetabolites, such as azathioprine and 6-mercaptopurine; (4) polyclonal and monoclonal anti-lymphocyte antibodies, such as anti-lymphocyte globulin and OKT3; and (5) alkylating agents, such as cyclophosphamide. In some specific embodiments, the immunosuppressant is, for example, methylprednisolone, prednisone, azathioprine, Prograf, Zenapax, Simulect, cyclosporine, tacrolimus, rapamycin, mycophenolate mofetil, mizoribine, cyclophosphamide, or fingolimod.

In some embodiments, the pharmaceutical composition further comprises at least one pharmaceutical auxiliary material.

The eleventh aspect of the present invention provides use of the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt in the preparation of a medicament for the prevention and/or treatment of a tumor.

The twelfth aspect of the present invention provides a method for treating and/or preventing a tumor, comprising: administering to a subject in need thereof a therapeutically and/or prophylactically effective amount of the aforementioned linker-drug conjugate, or the pharmaceutically acceptable salt or solvate thereof, or the solvate of the salt, or the aforementioned antibody-drug conjugate, or the pharmaceutically acceptable salt or solvate thereof, or the solvate of the salt.

The "tumor" as described in the present invention refers to a lesion formed by the excessive proliferation of local cells in an organ or tissue, including hematological tumors and solid tumors (including benign tumors and malignant tumors). In certain embodiments, the tumor is selected from tumors that are positive for or highly express the following markers: HER2 and EGFR.

In certain embodiments, the tumor is selected from: breast cancer, melanoma, meningioma, soft tissue sarcoma, salivary gland tumor, primary liver cancer, intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia, gastric cancer, nasopharyngeal cancer, laryngeal cancer, oral cancer, esophageal cancer, lung cancer (e.g., non-small cell lung cancer and small cell lung cancer), kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi sarcoma, non-melanoma skin cancer (including squamous cell carcinoma and basal cell carcinoma), hemangioma, glioma, and brain glioma.

### Preparation method

The thirteenth aspect of the present invention provides a method for preparing the aforementioned linker-drug conjugate, or the pharmaceutically acceptable salt or solvate thereof, or the solvate of the salt, comprising the following steps:
1. preparing a linker fragment X consisting of 1-4 amino acids;
2. linking X to a chemical structure M comprising a maleimide (m) fragment or a cyclooctyne fragment to obtain M-X; and
3. linking a cytotoxin D containing a (-NH₂) group to M-X to obtain the linker-drug conjugate M-X-D.

The above preparation method will be further illustrated by taking mc-AAN-Exatecan as an example below. The exemplary preparation process of mc-AAN-Exatecan is as follows:
1. activating Fmoc-Ala-OH with HOSu, followed by reacting with L-Ala to obtain Intermediate 1, wherein, the structural formula of Intermediate 1 is as shown below: 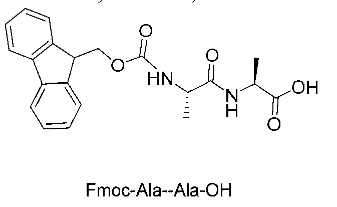
2. activating Intermediate 1 with HOSu, followed by reacting with L-Asn to obtain Intermediate 2, wherein, the structural formula of Intermediate 2 is as shown below: 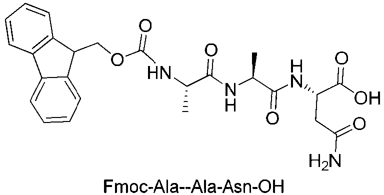
3. subjecting Intermediate 2 to a de-Fmoc reaction to obtain Intermediate 3, wherein, the structural formula of Intermediate 3 is as shown below: 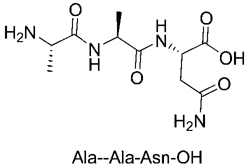
4. reacting Intermediate 3 with N-succinimidyl 6-maleimidohexanoate to obtain Intermediate 4, wherein, the structural formula of Intermediate 4 is as shown below: 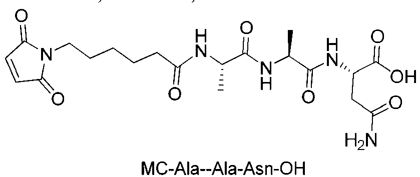
5. and condensing Intermediate 4 with Exatecan to obtain the product, wherein, the structural formula of the product is as shown below:

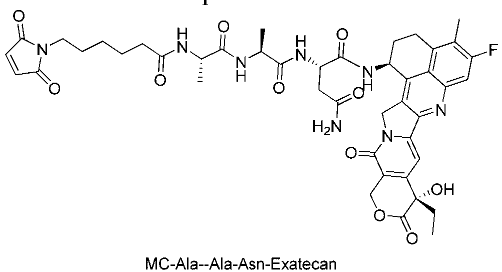

The fourteenth aspect of the present invention provides a method for preparing the aforementioned antibody-drug conjugate, or the pharmaceutically acceptable salt or solvate thereof, or the solvate of the salt, comprising the following steps:
1. taking a certain weight of a monoclonal antibody, diluting with PBS buffer, and adjusting the pH of the antibody to around 7.5; detecting the protein concentration, weighing the net weight of the antibody solution, and calculating the total protein amount; and adding a TCEP solution to the antibody, and reacting at room temperature while continuously shaking;
2. adding an excess of a cytotoxic drug solution to the reduced antibody solution, mixing evenly, and then reacting at room temperature while continuously mixing; and after the reaction is completed, adding an excess of N-acetylcysteine solution to the reaction mixture, and reacting at room temperature while continuously mixing; and
3. purifying the conjugated product using an ultrafiltration centrifuge tube, exchanging into a storage solution, and then filtering through a sterilizing filter to obtain the antibody-drug conjugate.

### Beneficial Effects

In certain embodiments, the linker provided in the present invention can release the Payload by means of an enzymatic hydrolysis reaction in the case of removing the hydrophobic structure PAB, which can not only reduce the hydrophobicity of the Linker-Drug and reduce the content of the polymers produced in the preparation of the ADC, but also can exhibit the corresponding cell killing activity.

### Definition of Terms

### Detailed Description of Embodiments

Embodiments of the present invention will be described in detail below with reference to examples; however, those skilled in the art will understand that the following examples are only used to illustrate the present invention and should not be regarded as limiting the scope of the present invention. For examples in which specific conditions are not specified, they are carried out according to the conventional conditions or the conditions recommended by the manufacturer. The reagents or instruments used without indicating the manufacturer are all commercially available conventional products.

In the present invention, the scientific and technical terms used herein have the meanings that are commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for better understanding of the present invention, definitions and explanations of related terms are provided below.

In the present invention, any numerical range should be interpreted as including any value within the range or any subrange unless otherwise specified.

In the present invention, the term "antibody" refers to an immunoglobulin molecule that generally consists of two pairs of identical polypeptide chains (each pair has one "light" (L) chain and one "heavy" (H) chain). Antibody light chains can be divided into two categories, i.e., κ and λ. Heavy chains can be divided into five types, i.e., µ, δ, γ, α, or ε. According to different heavy chains, antibodies can be divided into five categories, i.e., IgM, IgD, IgG, IgA, and IgE. Within light and heavy chains, the variable and constant regions are joined by a "J" region of about 12 or more amino acids, with the heavy chain further comprising a "D" region of about 3 or more amino acids. Each heavy chain consists of a heavy chain variable region (V_{H}) and a heavy chain constant region (C_{H}). The heavy chain constant region consists of three domains (C_{H}1, C_{H}2, and C_{H}3). Each light chain consists of a light chain variable region (V_{L}) and a light chain constant region (C_{L}). The light chain constant region consists of one domain, i.e., C_{L}. The constant regions of the antibodies can mediate the binding of the immunoglobulin to host tissues or factors, including various cells of the immune system (e.g., effector cells) and component C1q of the complement system. The V_{H} and V_{L} regions can be further subdivided into regions of hypervariability, termed complementarity determining regions (CDRs), interspersed with regions that are more conserved, termed framework regions (FRs). Each V_{H} and V_{L} consists of three CDRs and four FRs, arranged from amino-terminus to carboxy-terminus in the following order: FR1, CDR1, FR2, CDR2, FR3, CDR3, FR4. The variable regions (V_{H} and V_{L}) of each heavy/light chain pair form antibody binding sites, respectively. Distribution of amino acids in various regions or domains follows the definitions in: Kabat Sequences of Proteins of Immunological Interest (National Institutes of Health, Bethesda, Md. (1987 and 1991)), or Chothia & Lesk (1987) J. Mol. Biol. 196:901-917; and Chothia et al. (1989) Nature 342:878-883.

In certain embodiments, fewer than the theoretical maximum number of drug modules are conjugated to the antibody in the conjugation reaction. Generally, the antibody does not contain many free and reactive cysteine thiol groups, which can link drug modules; and in fact, most cysteine thiol groups in the antibody exist as disulfide bridges. In certain embodiments, the antibody can be reduced with a reducing agent such as dithiothreitol (DTT) or tris(2-carboxyethyl)phosphine (TCEP) under partial or complete reducing conditions to produce reactive cysteine thiol groups.

In the present invention, the term "pharmaceutically acceptable salt" refers to (i) salts formed from acidic functional groups present in the conjugate provided by the present invention and appropriate inorganic or organic cations (bases), including but not limited to alkali metal salts, such as sodium salts, potassium salts, and lithium salts; alkaline earth metal salts, such as calcium salts and magnesium salts; other metal salts, such as aluminum salts, iron salts, zinc salts, copper salts, nickel salts, and cobalt salts; inorganic base salts, such as ammonium salts; and organic base salts, such as tert-octylamine salts, dibenzylamine salts, morpholine salts, glucosamine salts, alkyl phenylglycinate salts, ethylenediamine salts, N-methylglucosamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenylethylamine salts, piperazine salts, tetramethylamine salts, and tris(hydroxymethyl)aminomethane salts; and (ii) salts formed from basic functional groups present in the conjugate provided by the present invention and appropriate inorganic or organic anions (acids), including but not limited to hydrohalides, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-benzenesulfonates; organic acid salts, such as acetates, malates, fumarates, succinates, citrates, tartrates, oxalates, and maleates; and amino acid salts, such as glycine salts, trimethylglycine salts, arginine salts, ornithine salts, glutamic acid salts, and aspartic acid salts.

Pharmaceutically acceptable salts can be obtained using standard procedures well known in the art, for example, by reacting a sufficient amount of a basic substance with a suitable acid providing a pharmaceutically acceptable anion, or by reacting a sufficient amount of an acidic substance with a suitable base providing a pharmaceutically acceptable cation.

In the present invention, a solvate refers to the following form of the antibody-drug conjugate of the present invention: a solid or liquid complex formed by coordination of the antibody-drug conjugate with solvent molecules. A hydrate is a specific form of solvate, which has coordinated water molecules. In the present invention, the hydrate is a preferred solvate.

Methods for preparing various pharmaceutical compositions containing certain amounts of active ingredients are known or apparent to those skilled in the art according to the invention of the present invention. As described in REMINGTON'S PHARMACEUTICAL SCIENCES, Martin, E.W., ed., Mack Publishing Company, 19th ed. (1995), a method for preparing the pharmaceutical composition comprises incorporating appropriate pharmaceutical excipients, carriers, diluents, etc., which are non-toxic to cells or mammals exposed thereto at the used dosage and concentration.

In the present invention, pharmaceutical auxiliary materials refer to excipients and additives used during drug production and prescription deployment, and refer to substances that have been reasonably evaluated in terms of safety except active ingredients and are included in pharmaceutical preparation. Pharmaceutical auxiliary materials not only form dosage forms, act as carriers, and enhance stability, but also have important functions such as solubilization, dissolution enhancement, and sustained and controlled release. They are important ingredients that may affect the quality, safety, and efficacy of drug products. According to the sources thereof, they can be divided into natural, semi-synthetic, and fully synthetic. According to the functions and uses thereof, they can be divided into: solvents, propellants, solubilizers, cosolvents, emulsifiers, colorants, binders, disintegrants, fillers, lubricants, wetting agents, osmotic pressure regulators, stabilizers, glidants, correctants, preservatives, suspending agents, coating materials, fragrances, anti-adhesives, antioxidants, chelating agents, penetration enhancers, pH regulators, buffers, plasticizers, surfactants, foaming agents, defoamers, thickeners, inclusion agents, humectants, absorbents, diluents, flocculants and deflocculants, filter aids, release retardants, etc. According to the routes of administration thereof, they can be divided into pharmaceutical auxiliary materials given via oral administration, injection, mucosal administration, transdermal or local administration, nasal or oral inhalation administration, ocular administration, etc. The same pharmaceutical auxiliary material can be used in pharmaceutical preparations for different routes of administration and have different functions and uses.

In the present invention, the pharmaceutical composition can be prepared into various suitable dosage forms according to the route of administration. Examples are tablets, capsules, granules, oral solutions, oral suspensions, oral emulsions, powders, tinctures, syrups, injections, suppositories, ointments, creams, pastes, ophthalmic preparations, pills, implants, aerosols, inhalation powders, sprays, etc. The pharmaceutical composition or suitable dosage form can contain 0.01 mg to 1000 mg of the antibody-drug conjugate, or the pharmaceutically acceptable salt or solvate thereof, or the solvate of the salt of the present invention.

As used herein, the term "treatment" generally refers to obtaining a desired pharmacological and/or physiological effect. The effect may be prophylactic on the basis of the complete or partial prevention of a disease or symptoms thereof; and/or the effect may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

In the present invention, a "subject" refers to a vertebrate. In certain embodiments, a vertebrate refers to a mammal. Mammals include, but are not limited to, livestock (such as cattle), pets (such as cats, dogs, and horses), primates, mice, and rats. In certain embodiments, a mammal refers to a human.

In the present invention, an "effective amount" refers to an amount effective, at dosages and for periods of time necessary, to achieve a desired therapeutic or prophylactic effect. A "therapeutically effective amount" of the substance/molecule of the present invention may vary depending on factors such as the disease state, age, sex and body weight of the individual and the ability of the substance/molecule to elicit a desired response in the individual. The therapeutically effective amount further covers an amount in which the therapeutically beneficial effect of the substance/molecule outweighs any toxic or harmful consequences. A "prophylactically effective amount" refers to an amount effective, at dosages and for periods of time necessary, to achieve a desired prophylactic effect. Usually but not necessarily, since the prophylactic dose is given to the subject before the onset of the disease or at the early stage of the disease, the prophylactically effective amount will be lower than the therapeutically effective amount. In the case of cancer, the therapeutically effective amount of a drug can reduce the number of cancer cells; reduce the tumor volume; inhibit (i.e., slow down to some extent, preferably stop) the infiltration of cancer cells into surrounding organs; inhibit (i.e., slow down to some extent, preferably stop) tumor metastasis; inhibit tumor growth to some extent; and/or alleviate one or more symptoms related to cancer to some extent.

In the present invention, 20 conventional amino acids and abbreviations thereof follow conventional usages. See Immunology-A Synthesis (2nd edition, E.S. Golub and D.R. Gren, Eds., Sinauer Associates, Sunderland, Mass. (1991)), which is incorporated herein by reference.

In the present invention, if X is Ala-Ala-Gln, it means that X is a linker fragment consisting of Ala, Ala, and Gln sequentially linked through the formation of peptide bonds. Moreover, those skilled in the art can understand that it does not limit the specific steric configurations of Ala, Ala, and Gln, such as the L-configuration or D-configuration. Similarly, if X is Ala-Ala-(3-cyano-alanine), it means that X is a linker fragment consisting of Ala, Ala, and (3-cyano-alanine) sequentially linked through the formation of peptide bonds. Moreover, those skilled in the art can also understand that it does not limit the specific steric configurations of Ala, Ala, and (3-cyano-alanine), such as the L-configuration or D-configuration, or the S-configuration or R-configuration. Other similar limitations of X in the present invention can be understood by referring to the aforementioned content.

In the present invention, if X is selected from Ala-Ala-Gln, Ala-Ala-(3-cyano-alanine), and their deuterated compounds, it means that X can be Ala-Ala-Gln, or Ala-Ala-(3-cyano-alanine), or the deuterated compound of Ala-Ala-Gln, or the deuterated compound of Ala-Ala-(3-cyano-alanine).

The term "deuterated compound" refers to a compound formed by replacing one or more (such as 2, 3, 4 or more) hydrogen atoms in a certain compound (the positions of the hydrogen atoms are not specifically limited, and they can be hydrogen atoms on an alkyl group, or hydrogen atoms on an amino group, etc.) with deuterium atoms.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a non-human (e.g., mouse) antibody format, which is a chimeric immunoglobulin, immunoglobulin chain, or a fragment thereof (e.g., Fv, Fab, Fab', F(ab')2, or other antigen-binding subsequences of the antibody) and contains minimal sequence derived from non-human immunoglobulin. Preferably, the humanized antibody is a human immunoglobulin (recipient antibody) in which a residue in the complementarity determining region (CDR) of the recipient antibody is replaced by a CDR residue from a non-human species (donor antibody) such as mouse, rat, or rabbit having the desired specificity, affinity, and capacity.

In addition, during humanization, it is also possible to mutate amino acid residues in CDR1, CDR2 and/or CDR3 regions of VH and/or VL, thereby improving one or more binding properties (e.g., affinity) of the antibody. Mutations can be introduced, for example, by PCR-mediated mutations, the effect of which on antibody binding or other functional properties can be evaluated using *in vitro* or *in vivo* assays as described herein. Generally, conservative mutations are introduced. Such mutations may be amino acid substitutions, additions or deletions. In addition, there are generally no more than one or two mutations within a CDR.

The present invention will be further explained with reference to specific examples below, and these examples do not limit the scope of the present invention.

### Example 1. Preparation of the linker drug MCC-AAQ-Exatecan

### 1. Synthesis of Intermediate 1, Fmoc-Gln-Exatecan

Fmoc-Gln-OH (N-fluorenylmethyloxycarbonyl-L-glutamine) (76.3 mg, 1.1 eq.) and Exatecan (100 mg, 1.0 eq.) were added into a reaction flask, followed by DMF (1 ml), DIEA (29 mg, 1.5 eq.), and TBTU (72.5 mg, 1.1 eq.), and the reaction was carried out at room temperature for 2 hours. The reaction was monitored by HPLC until no starting materials remained. After the reaction was completed, purification was carried out by medium-pressure column chromatography (DCM/MeOH). The product was collected and concentrated to obtain Intermediate 1.

The reaction formula was as shown below:

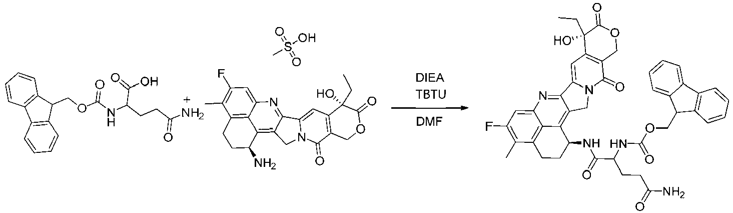

### 2. Synthesis of Intermediate 2, Gln-Exatecan

Intermediate 1, Fmoc-Gln-Exatecan (130 mg, 1.0 eq.) was added into a reaction flask, followed by DCM (2 ml). The mixture was stirred at room temperature. DEA (0.5 ml) was added, and the reaction was carried out at room temperature. After 4 hours, the reaction was monitored by HPLC until no starting materials remained. The reaction was stopped. MTBE (10 ml) was slowly added, so that a large amount of solid was precipitated out, and the mixture was stirred for 30 min. The solid was filtered and washed with MTBE to obtain an off-white solid, which was dried to obtain 95 mg of Intermediate 2.

The reaction formula was as shown below:

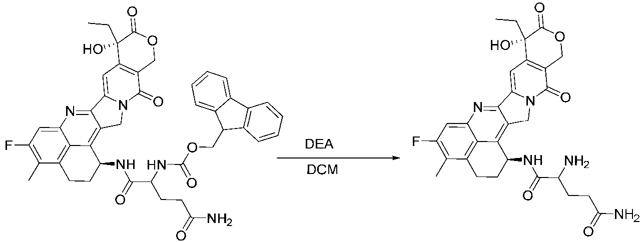

### 3. Synthesis of Intermediate 3, Fmoc-Ala-Ala-Gln-Exatecan

Intermediate 2, Gln-Exatecan (100 mg, 1.0 eq.) was added into a reaction flask, followed by DMF (1 mL), Fmoc-Ala-Ala-OH (67.8 mg, 1.0 eq.), TBTU (68.4 mg, 1.2 eq.), and DIEA (34.4 mg, 1.5 eq.), and the reaction was carried out at room temperature for 3 hours. The reaction was monitored by HPLC until it was completed. Purification was carried out by medium-pressure column chromatography (DCM/MeOH). The product was collected to obtain Intermediate 3.

The reaction formula was as shown below:

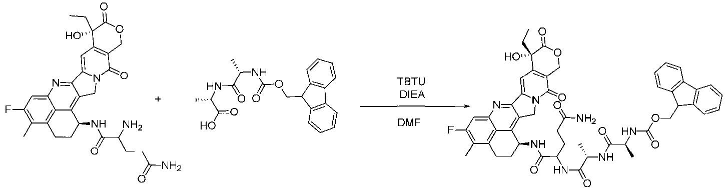

### 4. Synthesis of Intermediate 4, Ala-Ala-Gln-Exatecan

Intermediate 3, Fmoc-Ala-Ala-Gln-Exatecan (140 mg, 1.0 eq.) was added into a reaction flask, followed by DCM (2 ml). The mixture was stirred at room temperature. DEA (0.5 ml) was added, and the reaction was carried out at room temperature. After 4 hours, the reaction was monitored by HPLC until it was completed. MTBE (10 ml) was slowly added, so that a large amount of solid was precipitated out, and the mixture was stirred for 30 min. The solid was filtered and washed with MTBE to obtain an off-white solid, which was dried to obtain Intermediate 4.

The reaction formula was as shown below:

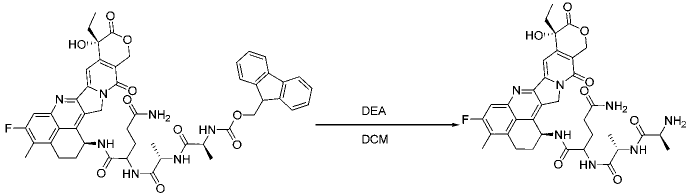

### 5. Synthesis of the product, MCC-Ala-Ala-Gln-Exatecan

Intermediate 4, Ala-Ala-Gln-Exatecan (50 mg, 1.0 eq.) was added into a reaction flask, followed by DMF (1 mL), MCC (18 mg, 1.1 eq.), HATU (80.8 mg, 3.0 eq.), and DIEA (36.6 mg, 4.0 eq.). After the reaction was carried out at room temperature for 2 hours, it was monitored by HPLC until no Intermediate 4 remained, and the reaction was stopped. Preparation and purification were carried out by Prep-HPLC. The product was collected and concentrated to dryness to obtain the product (the NMR spectrum was as follows).

The reaction formula was as shown below:

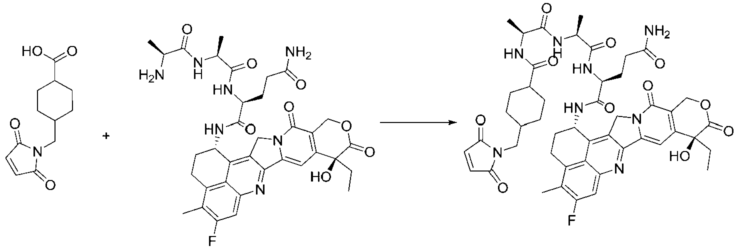

¹H NMR (400 MHz, DMSO-*d*₆) δ 8.17-8.26 (d, *J* =2.0 Hz, 1H), 7.83-7.91 (d, *J* =2.0 Hz, 2H), 7.65-7.74(d, *J* =2.0 Hz, 2H), 7.21-7.28(d, *J* =8.0 Hz,2H), 6.98-7.04(m,2H), 5.43-5.50(m,1H), 5.36-5.43(m,2H), 5.15-5.24(m,1H), 5.01-5.11(m,1H), 4.14-4.24(m,1H), 3.97-4.08(m,1H), 3.47-3.57(m,1H), 3.20-3.27(m,1H), 3.05-3.16(m,2H), 2.30-2.36(m,2H), 2.00-2.15(m,4H), 1.77-1.92(m,3H), 1.65-2.00(m,5H), 1.45-1.64(m,4H), 1.09-1.24(m,5H), 0.95-1.07(m,4H), 0.79-0.94(m,5H).

### Example 2. Preparation of the linker drug MCC-Ala-Ala-Asn-Exatecan

### 1. Synthesis of Intermediate 1

After Fmoc-Ala-Ala-OH (N-[fluorenylmethyloxycarbonyl]-L-alanyl-L-alanine) was activated with HOSu (N-hydroxysuccinimide), it was reacted with L-Asn (L-asparagine) to obtain Intermediate 1.

The specific steps were as follows: Fmoc-Ala-Ala-OH (1.0 eq.), HOSu (1.3 eq.), and THF (10 v/w) were added into a reaction flask at room temperature. DCC (1.3 eq.) was slowly added under stirring while controlling the temperature at room temperature. The reaction was carried out at room temperature. The reaction was monitored by HPLC until the residual amount of Fmoc-Ala-Ala-OH was low (using the area normalization method). The reaction mixture was filtered, and the filter cake was rinsed with THF (2.5 v/w). Purified water (11.5 v/w) was added into the filtrate, followed by L-Asn (1.1 eq.) and solid sodium bicarbonate (1.0 eq.), and the mixture was stirred and reacted at room temperature. The reaction was monitored by HPLC until the residual amount of Fmoc-Ala-Ala-OSu was low. Then, citric acid monohydrate (1.0 eq.) was added, and the mixture was stirred. The reaction mixture was concentrated to remove most of the solvent. DMF (4 v/w) was added to dissolve the product. After filtering, reverse-phase preparation was carried out by preparative liquid chromatography. After the preparative solution was concentrated until no obvious droplets flowed out, it was extracted with ethyl acetate four times. The ethyl acetate phase was concentrated to dryness to obtain Intermediate 1. The reaction formula was as shown below:

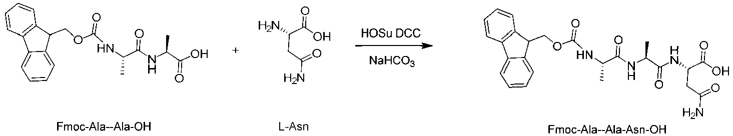

### 2. Synthesis of Intermediate 2

After the Fmoc group was removed from Intermediate 1 by adding DEA (diethylamine), Intermediate 2 was obtained.

The specific steps were as follows: Intermediate 1 (1.0 eq.) was added into a reaction flask at room temperature, followed by DMF (15 v/w). DEA (3 v/w) was added dropwise while controlling the temperature at room temperature. The reaction was carried out at room temperature. The reaction was monitored by HPLC until no Intermediate 1 remained. DMF was removed by concentration. DCM (40 v/w) and purified water (40 v/w) were added. After stirring, liquid separation was carried out. The aqueous phase was concentrated to dryness to obtain Intermediate 2. The reaction formula was as shown below:

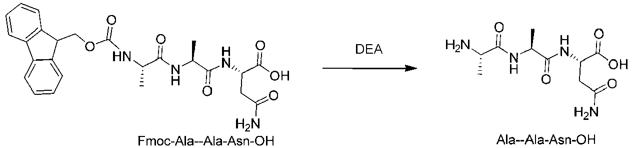

### 3. Synthesis of Intermediate 3

Intermediate 2 was reacted with N-succinimidyl 4-(N-maleimidomethyl) cyclohexanecarboxylate to obtain Intermediate 3.

The specific steps were as follows: Intermediate 2 (1.0 eq.) and N-succinimidyl 4-(N-maleimidomethyl)cyclohexanecarboxylate (1.1 eq.) were added into a reaction flask at room temperature, followed by DMF (22 v/w), water (9.5 v/w), and DIPEA (1.0 eq.). Then, the reaction was carried out at room temperature until the residual amount of Intermediate 2 was low (detected by QDA). After the reaction was completed, most of the solvent was removed by concentration. Water (50 v/w) and EA (50 v/w) were added, followed by 1.0 eq. of DIEA. After the mixture was stirred until dissolved and clarified, it was left to stand for liquid separation. The aqueous phase was extracted twice with EA (50 v/w * 2), and the aqueous phase was concentrated to dryness to obtain Intermediate 3. The reaction formula was as shown below:

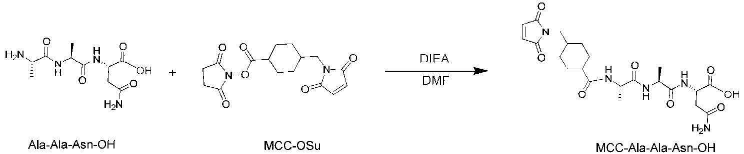

### 4. Synthesis of the product

Intermediate 3 was subjected to a condensation reaction with exatecan mesylate (CAS No.: 169869-90-3) using HATU to obtain Intermediate 4.

The specific steps were as follows: Intermediate 3 (1.2 eq.) was added into a reaction flask at room temperature, followed by DMF (15 v/w). Exatecan mesylate (1.0 eq.), TBTU (1.4 eq.), and DIPEA (4.5 eq.) were added successively. Then, the reaction was carried out at room temperature, and in-process monitoring was carried out by HPLC. After the reaction was completed, purification was carried out by prep-HPLC. The preparative solution was concentrated, extracted with DCM, and concentrated to obtain the product (the NMR spectrum was as follows). The reaction formula was as shown below:

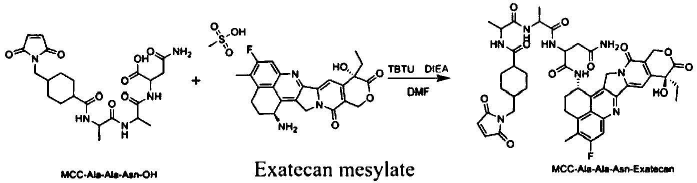

¹H NMR (400 MHz, DMSO-*d*₆) δ 8.15-8.25 (d, *J* =2.0 Hz, 1H), 7.85-8.0 (d, *J* =7.5Hz, 2H), 7.7-7.85(d, *J* =2.0 Hz, 1H), 7.33-7.4 (d, *J* =8.0 Hz,1H), 7.28-7.32 (m,1H), 6.98-7.05(d, *J* =8.0 Hz,2H), 6.85-6.95(m,1H), 6.45-6.57(m,1H), 5.45-5.55(m,1H), 5.40-5.45(m,1H), 5.20-5.27(m,1H), 4.40-4.50(m,1H), 3.95-4.05(m,1H), 3.20-3.27(m,2H), 3.10-3.18(m,2H), 2.86-2.97(m,2H), 2.36-2.43(m,3H), 2.15-2.25(m,1H), 1.95-2.10(m,3H), 1.80-1.93(m,3H), 1.73-1.78(m,1H), 1.55-1.70(m,3H), 1.45-1.55(m,3H), 1.0-1.3(m,10H), 0.80-0.95(m,5H).

### Example 3. Preparation of the linker drug MCC-Ala-Ala-(3-cyano-L-alanine)-Exatecan

### 1. Synthesis of Intermediate 1, (3-cyano-L-alanine)-Exatecan

Fmoc-Asn-OH (40 mg, 1.2 eq.) was added into a reaction flask, followed by DMF (1.5 ml), TBTU (45 mg, 1.5 eq.), and DIEA (60 mg, 2.5 eq.), and the mixture was stirred until it was completely dissolved. After stirring for 30 min, exatecan mesylate (50 mg, 1.0 eq.) was added, and the mixture was stirred for 4 hrs. The reaction was monitored by HPLC until it was stopped. Additional TBTU (45 mg, 1.5 eq.) and DIEA (60 mg, 2.5 eq.) were added, and the reaction was continued at room temperature. The reaction was monitored by HPLC until no starting material Exatecan remained, and Fmoc-(3-cyano-L-alanine)-Exatecan accounted for approximately 70%. The reaction was stopped, and preparation and purification were carried out by medium-pressure chromatography. The product was collected, and concentrated to dryness under reduced pressure at 30°C to obtain the product.

The above product was added into a reaction flask, followed by DCM (4 ml), and the mixture was stirred. DEA (1 ml) was added, and the mixture was stirred at room temperature until it was completely dissolved. The reaction was monitored by HPLC until no starting materials remained. 10 ml of MTBE was added, and the mixture was stirred at room temperature. A large amount of solid was precipitated out, and filtered to obtain an off-white solid. The filter cake was washed with MTBE and dried to obtain 35 mg of a solid, namely Intermediate 1.

The reaction formula was as shown below:

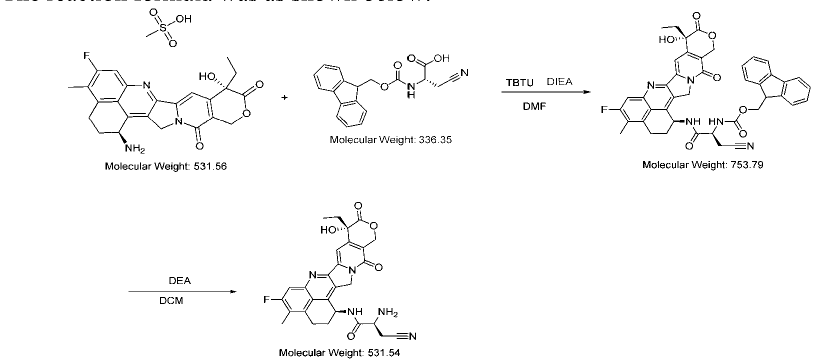

### 2. Synthesis of Intermediate 2, MCC-Ala-Ala-OH

SMCC (460 mg, 1.1 eq.) was added into a reaction flask, followed by NH₂-Ala-Ala-OH (200 mg, 1.0 eq.) and DMF (4 ml, 20 V), and the mixture was stirred. Then, DIPEA (161.4 mg, 1.0 eq.) was added. The mixture was heated to 60°C for reaction. As the reaction proceeded, the reaction mixture was gradually dissolved and clarified. The reaction was carried out for at least 8 hours. A sample was taken for LC-MS detection. After the reaction was completed, the reaction mixture was cooled down to room temperature. DMF was removed by concentration under reduced pressure at 35°C. Then, purified water (4 ml, 20 V) and DIPEA (161.4 mg, 1.0 eq.) were added. EA (4 ml, 20 V) was added, and the mixture was stirred and washed. The organic phase was discarded. Then, EA (2 ml, 10 V) was added for extraction twice. The organic phase was discarded. The aqueous phase was retained, and the acidity was adjusted to pH 1-2 with 2 M hydrochloric acid. A large amount of white solid was precipitated out. The mixture was stirred for one hour, and filtered to obtain a white solid. The solid was washed with purified water, and dried to obtain 320 mg of a white solid, namely Intermediate 2.

The reaction formula was as shown below:

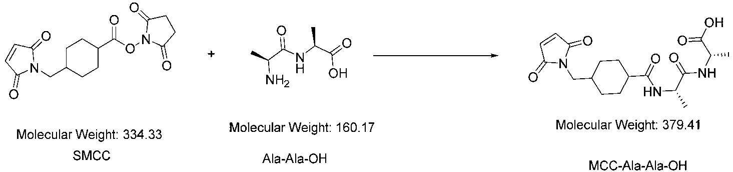

### 3. Synthesis of the product, MCC-Ala-Ala-(3-cyano-L-alanine)-Exatecan

Intermediate 1, (3-cyano-L-alanine)-Exatecan (35 mg, 1.0 eq.) was added into a reaction flask, followed by DMF (1 mL), Intermediate 2, MCC-Ala-Ala-OH (28 mg, 1.1 eq.), TBTU (25.3 mg, 1.2 eq.), and DIPEA (12.8 mg, 1.5 eq.). After the reaction was carried out at room temperature for 2 hours, it was monitored by HPLC until no Intermediate 1 remained, and the reaction was stopped. Purification was carried out by Prep-HPLC. The product was collected and concentrated to dryness to obtain a yellow glassy solid, namely the product (the NMR spectrum was as follows).

The reaction formula was as shown below:

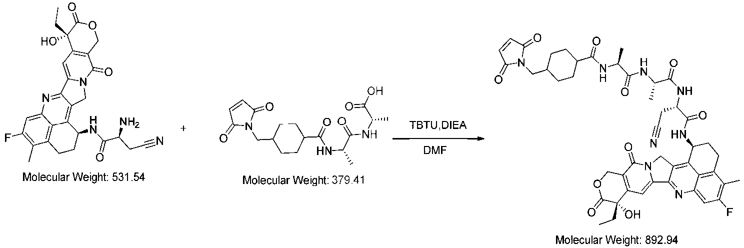

¹H NMR (400 MHz, DMSO-*d*₆) δ 8.45-8.55 (d, *J* =2.0 Hz, 1H), 8.23-8.29 (d, *J* =2Hz, 2H), 7.95-8.03(d, *J* =2.0 Hz, 1H), 7.84-7.92 (d, *J* =8.0 Hz,1H), 7.76-7.84 (m,1H), 7.27-7.33(d, *J* =8.0 Hz,1H), 6.98-7.02(m,1H), 5.47-5.56(m,1H), 5.40-5.45(m,1H), 5.26-5.36(m,1H), 5.1-5.2(m,1H), 4.44-4.55(m,1H), 4.07-4.19(m,1H), 3.88-4.0(m,2H), 3.19-3.26(m,2H), 3.13-3.18(m,1H), 2.90-3.0(m,2H), 2.76-2.88(m,2H), 2.36-2.43(m,3H), 2.00-2.12(m,2H), 1.80-1.93(m,3H), 1.80-1.92(m,2H), 1.55-1.70(m,3H), 1.13-1.26(m,5H), 1.05-1.12(m,3H), 0.80-0.95(m,5H).

### Example 4. Synthesis route of the linker drug MCC-Ala-Ala-((S)-2-amino-4-cyanobutanoic acid)-Exatecan

### 1. Synthesis of Intermediate 1, ((S)-2-amino-4-cyanobutanoic acid)-Exatecan

(S)-2-((((9H-fluoren-9-yl)methoxy)carbonyl)amino)-4-cyanobutyric acid (79.8 mg, 1.1 eq.) and exatecan mesylate (110 mg, 1.0 eq.) were added into a reaction flask, followed by DMF (1.5 ml), TBTU (79.73 mg, 1.5 eq.), and DIEA (80 mg, 2.5 eq.), and the reaction was carried out at room temperature for 3 hours. The reaction was monitored by HPLC. After the reaction was completed, 9 ml of ACN was added, and the mixture was stirred at room temperature. Then, 1.0 ml of DEA was added, and the reaction was continued at room temperature for 1 hour. A sample was taken for monitoring until no starting materials remained. 10 ml of MTBE was added, and the mixture was stirred until no obvious solid was precipitated out. After stirring for 1 hour, there was no obvious change.

The solvent was removed by concentration under reduced pressure at 30°C. During the concentration process, a large amount of solid was precipitated out. At this time, only a small amount of the solvent, approximately 3 ml, was removed. The concentration was stopped, and the mixture was continuously stirred at room temperature for 1 hour. After being filtered, the solid was washed with MTBE, and dried to obtain an off-white solid, namely Intermediate 1.

The reaction formula was as shown below:

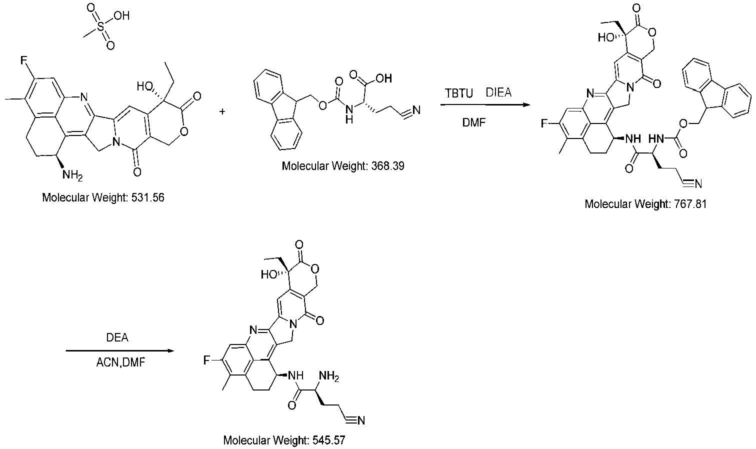

### 2. Synthesis of Intermediate 2, MCC-Ala-Ala-OH

SMCC (460 mg, 1.1 eq.) was added into a reaction flask, followed by NH₂-Ala-Ala-OH (200 mg, 1.0 eq.) and DMF (4 ml, 20 V), and the mixture was stirred. Then, DIPEA (161.4 mg, 1.0 eq.) was added. The mixture was heated to 60°C and reacted for 8 hours. A sample was taken for LC-MS detection. After the reaction was completed, the reaction mixture was cooled down to room temperature. DMF was removed by concentration under reduced pressure at 35°C. Then, purified water (4 ml, 20 V) and DIPEA (161.4 mg, 1.0 eq.) were added. EA (4 ml, 20 V) was added, and the mixture was stirred and washed. The organic phase was discarded. Then, EA (2 ml, 10 V) was added for washing twice. The organic phase was discarded. The aqueous phase was retained, and the acidity was adjusted to pH 1-2 with 2 M hydrochloric acid. A large amount of white solid was precipitated out. The mixture was stirred for one hour, and filtered to obtain a white solid. The solid was washed with purified water, and dried to obtain 320 mg of a white solid, namely Intermediate 2.

The reaction formula was as shown below:

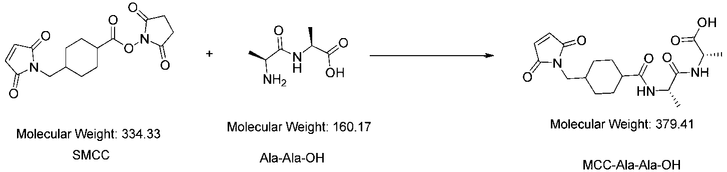

### 3. Synthesis of the product, MCC-Ala-Ala-((S)-2-amino-4-cyanobutanoic acid)-Exatecan

Intermediate 1, ((S)-2-amino-4-cyanobutanoic acid)-Exatecan (25 mg, 1.0 eq.) was added into a reaction flask, followed by DMF (1 mL), Intermediate 2, MCC-Ala-Ala-OH (19 mg, 1.1 eq.), TBTU (17.7 mg, 1.2 eq.), and DIPEA (8.88 mg, 1.5 eq.). After the reaction was carried out at room temperature for 2 hours, it was monitored by HPLC until it was completed. Preparation and purification were carried out by Prep-HPLC. The product was collected and concentrated to remove most of the acetonitrile. The resultant was lyophilized to obtain a yellow solid, namely the product (the NMR spectrum was as follows).

The reaction formula was as shown below:

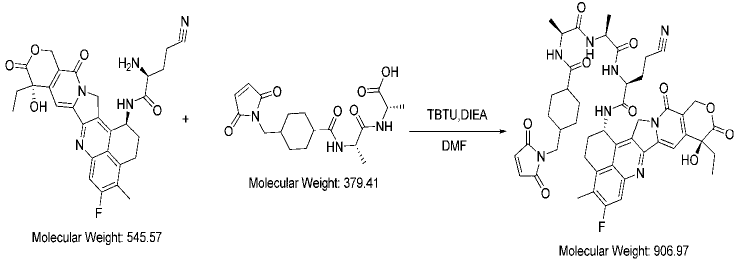

¹H NMR (400 MHz, DMSO-*d*₆) δ 8.30-8.37 (d, *J* =2.0 Hz, 1H), 7.87-7.96 (d, *J* =2.0 Hz, 2H), 7.75-7.86(d, *J* =2.0 Hz, 2H), 7.27-7.33(d, *J* =8.0 Hz,1H), 6.98-7.02(m,2H), 5.45-5.54(m,1H), 5.40-5.45(m,2H), 5.23-5.32(m,1H), 5.04-5.13(m,1H), 4.25-4.37(m,1H), 3.97-4.09(m,1H), 3.56-3.66(m,4H), 3.10-3.26(m,4H), 2.35-2.46(m,4H), 2.00-2.13(m,3H), 1.77-1.92(m,3H), 1.66-1.75(m,2H), 1.56-1.64(m,3H), 1.45-1.53(m,1H), 1.12-1.26(m,4H), 1.01-1.13(m,4H), 0.80-0.94(m,4H).

### Example 5. Synthesis route of the linker drug MCC-Ala-Ala-Ala-Ala-Gln-Exatecan

### 1. Synthesis of Intermediate 1, MCC-Ala-Ala-Ala-Ala-Gln-OH

H-Gln(Trt)-OH 2-CTC resin (1.0 g, 0.5 mmol, 0.5 mmol/g) was added into a solid-phase reactor, followed by DCM (20 mL). The resin was shaken in a shaker for 20 min to be fully swollen. The resin was filtered and washed with DMF three times. 20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF six times. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF six times to obtain H-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF six times. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF six times to obtain H-Ala-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF six times. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF six times to obtain H-Ala-Ala-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF six times. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF six times to obtain H-Ala-Ala-Ala-Ala-Gln (Trt)-2-CTC-resin.

20 mL of a DMF solution containing SMCC (3.0 eq., 501 mg, 1.5 mmol) was added into a reaction tube containing H-Ala-Ala-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. After being filtered, the resin was washed with DMF six times, MeOH three times, and MTBE three times successively. The resin was dried in a vacuum drying oven at 30°C for 1 hr to obtain MCC-Ala-Ala-Ala-Ala-Gln(Trt)-2-CTC-resin (1.42 g, 0.5 mmol).

The resulting resin above was transferred to a 50 mL centrifuge tube. A cold 95% TFA/Tis solution (15 mL) was added, and the reaction was carried out in a shaker for 1 hr. After filtering, the filtrate was slowly added into a cold MTBE solution (150 mL). The mixture was left to stand for 1 hr, and a white precipitate was precipitated out. The precipitate was centrifuged and washed with a cold MTBE solution three times. The washed precipitate was collected and dried in a vacuum drying oven at 30°C for 4 hrs to obtain a white solid, namely Intermediate 1, MCC-Ala-Ala-Ala-Ala-Gln-OH.

The reaction formula was as shown below:

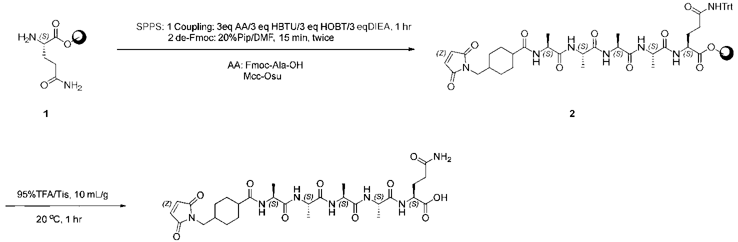

### 2. Synthesis of the product, MCC-Ala-Ala-Ala-Ala-Gln-Exatecan

Intermediate 1, MCC-Ala-Ala-Ala-Ala-Gln-OH (244 mg, 2.0 eq.) was added into a reaction flask, followed by DMF (2 mL), irinotecan mesylate (100 mg, 1.0 eq.), TBTU (132 mg, 2.2 eq.), and DIPEA (73 mg, 3.0 eq.). The mixture was stirred and reacted at room temperature for 1 hour. The reaction was monitored by HPLC until 10% of Exatecan remained.

Purification was carried out by Prep-HPLC. The product was collected. The preparative solution was concentrated to dryness to obtain a yellow solid (the NMR spectrum was as follows).

The reaction formula was as shown below:

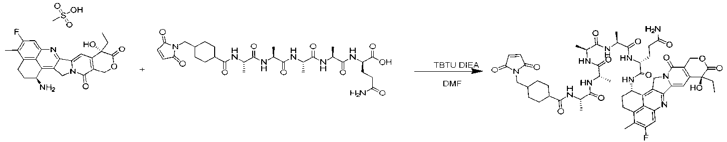

¹H NMR (400 MHz, DMSO-*d*₆) δ 8.25-8.33(d, *J* =2.0 Hz, 1H), 7.87-7.95 (d, *J* =2.0 Hz, 2H), 7.76-7.86(d, *J* =2.0 Hz, 2H), 7.28-7.34(d,*J* =8.0 Hz,1H), 7.20-7.26(m,1H), 6.98-7.02(m,1H), 6.73-6.76(m,1H), 5.47-5.55(m,1H), 5.40-5.41(m,1H), 5.26-5.36(m,2H), 5.05-5.15 (m,2H), 4.12-4.20(m,2H), 4.03-4.09(m,1H), 3.87-3.94(m,1H), 3.20-3.27(m,2H), 3.12-3.18(m,1H), 2.65-2.68(m,1H), 2.36-2.43(m,2H), 2.31-2.35(m,1H), 2.02-2.13(m,2H), 1.78-1.93(m,3H), 1.6-1.76(m,2H), 1.56-1.64(m,2H), 1.21-1.27(m,1H), 1.06-1.20(m,6H), 0.85-0.94(m,3H).

### Example 6: Synthesis route of the linker drug MCC-Ala-Ala-Gln-Belotecan

### Synthesis route of MCC-Ala-Ala-Gln-Belotecan

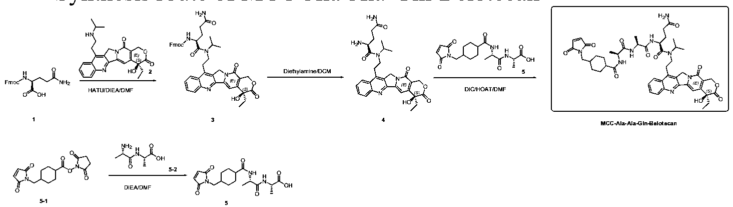

### 1. Synthesis of Intermediate 3, Fmoc-Gln-Belotecan

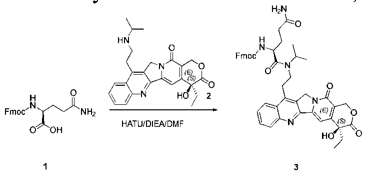

Intermediate 1 (212 mg, 0.575 mmol, 5.0 eq.), Belotecan (50 mg, 0.115 mmol, 1.0 eq.), and HATU (219 mg, 0.575 mmol, 5 eq.) were added into a reaction flask, followed by DMF (3 mL), and the mixture was shaken to dissolve it. DIEA (190 µL, 1.15 mmol, 10 eq.) was added at 10°C, and the mixture was stirred and reacted at 10°C for 1 hr. The reaction was monitored by HPLC until Belotecan was reacted completely. Preparation and purification were carried out. The product was collected, and concentrated and lyophilized to obtain a yellow solid product 3, Fmoc-Gln-Belotecan (51 mg, purity: 98.71%, yield: 55.84%). MS(ESI): m/z 784.3[M+H]⁺.

### 2. Synthesis of Intermediate 4, Gln-Belotecan

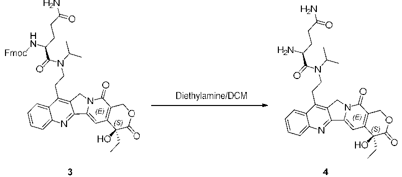

Intermediate 3, Fmoc-Gln-Belotecan (51 mg, 0.065 mmol, 1.0 eq.) was added into a reaction flask, followed by DMF (4 mL) and diethylamine (1 mL), and the mixture was stirred at 20°C for 2 hrs. The reaction was monitored by HPLC until no starting materials remained. The reaction mixture was concentrated in vacuo. DMF (2 mL) was added. After preparation and purification, the preparative solution was concentrated and then lyophilized to obtain a yellow solid 4, Gln-Belotecan (24 mg, purity: 91.96%, yield: 60.40%). MS(ESI): m/z 562.34[M+H]⁺.

### 3. Synthesis of Intermediate 5, MCC-Ala-Ala-OH

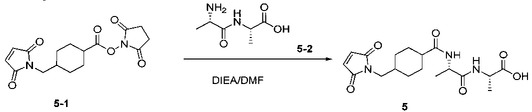

The starting materials 5-1, MCC-OSu (9.5 g, 59.31 mmol, 1.0 eq.) and 5-2, Ala-Ala-OH (21.8 g, 65.24 mmol, 1.1 eq.) were added into a reaction flask. DMF (190 mL) was added at 25°C, followed by DIEA (9.8 mL, 59.31 mmol, 1.0 eq.), and the mixture was stirred and reacted at 60°C for 8 hrs. The reaction was monitored by HPLC until it was completed. The reaction mixture was cooled down to 25°C and concentrated in vacuo. Then, H₂O (190 mL), DIEA (9.8 mL), and EA (190 mL) were added successively. After being stirred at 25°C for 1 hr, the mixture was added into a separating funnel and left to stand for liquid separation. The aqueous phase was separated out and extracted twice with EA (95 mL). The EA layer was removed. A 2 M hydrochloric acid solution (71.3 mL) was slowly added dropwise to the aqueous phase. After being stirred at 25°C for 1 hr, the mixture was filtered. The resulting solid was dried under vacuum to obtain a white solid 5, MCC-Ala-Ala-OH (16.55 g, purity: 95%, yield: 73.54%). MS(ESI): m/z 380.2[M+H]⁺.

### 4. Synthesis of MCC-Ala-Ala-Gln-Belotecan

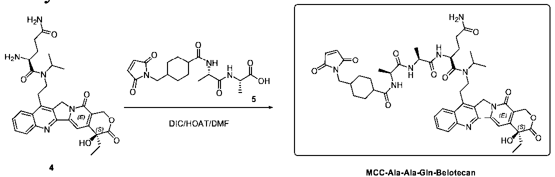

Intermediate 4 (15 mg, 0.027 mmol, 1.0 eq.), Intermediate 5, MCC-Ala-Ala-OH (15.2 mg, 0.04 mmol, 1.5 eq.), and HOAT (7.3 mg, 0.054 mmol, 2.0 eq.) were added into a reaction flask, followed by DMF (2 mL), and the mixture was shaken to dissolve it. DIC (8.0 µL, 0.054 mmol, 2.0 eq.) was added at 10°C, and the mixture was stirred and reacted at 20°C for 3 hrs. The reaction was monitored by HPLC until Intermediate 4 was reacted completely. Preparation and purification were carried out. The product was collected, and concentrated and lyophilized to obtain a yellow solid product, MCC-Ala-Ala-Gln-Belotecan (11 mg, purity: 96.42%, yield: 43.03%). MS(ESI): m/z 462.5[M+2H]/2⁺.

¹H NMR (400 MHz, DMSO-*d*₆) δ 8.58 (d, *J=* 10.4 Hz, 1H), 8.19 (d, *J=* 8.0 Hz, 1H), 8.15 (d, *J=* 8.0 Hz, 1H), 7.92-7.87 (m, 4H), 7.78 (t, *J=* 8.0 Hz, 1H), 7.35 (s, 1H), 7.21 (brs, 1H), 6.99 (s, 2H), 6.77 (brs, 1H), 5.46-5.43 (m, 4H), 4.79-4.76 (m, 1H), 4.35-4.28 (m, 1H), 4.26-4.21 (m, 2H), 3.42-3.38 (m, 2H), 3.23 (d, *J=* 6.8 Hz, 2H), 3.22-3.18 (m, 1H), 2.22-2.07 (m, 3H), 1.93-1.84 (m, 3H), 1.75-1.66 (m, 3H), 1.62-1.59 (m, 2H), 1.27-1.26 (m, 4H),1.23-1.21 (m, 5H), 1.18-1.16 (m, 6H), 0.90-0.86 (m, 6H).

In addition, those skilled in the art could also refer to the preparation methods of Example 1 to Example 6 above to obtain other linker-drug conjugates of the present invention, for example, MCC-AAQ-Rapamycin and

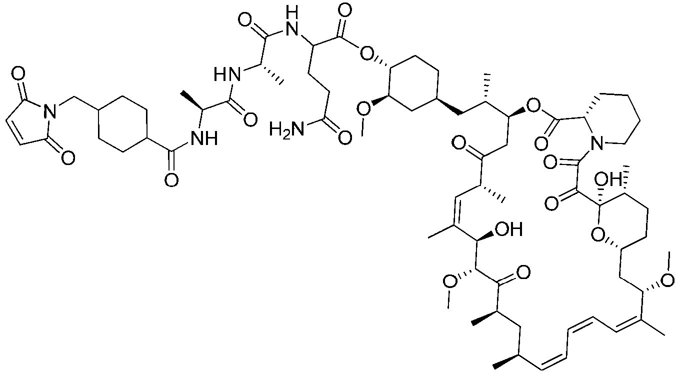

### MCC-AAQ-Rapamycin.

### Example 7. Preparation of the EGFR antibody-drug conjugate

### I. Preparation of the antibody conjugate EGFR antibody-MCC-AAQ-Exatecan

1. An EGFR antibody was taken, and its pH was adjusted to around 7.5 with a Tris-EDTA solution. The protein concentration was detected using Nanodrop, the net weight of the antibody solution was weighed, and the total protein amount was calculated. A TCEP solution was added into the antibody, and the mixture was placed on a 3D shaker and reacted at room temperature for more than 120 min with continuous mixing to completely reduce the interchain disulfide bonds of the antibody.
2. An excess of MCC-AAQ-Exatecan solution (dissolved in DMSO) was added into the reduced antibody solution. After being mixed evenly, the mixture was placed on a 3D shaker and reacted at room temperature for more than 30 min with continuous mixing. After the reaction was completed, an excess of N-acetylcysteine solution was added into the reaction mixture. The mixture was placed on a 3D shaker and reacted at room temperature for more than 30 min with continuous mixing.
3. The conjugated product was purified using a 30KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody-drug conjugate, EGFR antibody-MCC-AAQ-Exatecan, which was stored at 4°C.
4. The protein concentration of the antibody-drug conjugate was detected by the UV/BCA method, the DAR was detected by HIC, and the purity was detected by SEC.

### II. Preparation of other EGFR antibody-drug conjugates

For the method for preparing an antibody-drug conjugate by using other linker-drug conjugates of the present invention (such as the linker-drug conjugate as shown in formula I) and an EGFR antibody, reference may be made to the preparation method of the above antibody conjugate EGFR antibody-MCC-AAQ-Exatecan.

### Example 8. Preparation of the HER2 antibody-drug conjugate

For the method for preparing an antibody-drug conjugate (such as the antibody-drug conjugate HER2 antibody-MCC-AAN-Exatecan) by using the linker-drug conjugate of the present invention (such as the linker-drug conjugate as shown in formula I) and a HER2 antibody, reference may be made to the preparation method of the antibody conjugate EGFR antibody-MCC-AAQ-Exatecan in Example 7 above.

### Example 9. In vitro activity testing of various antibody-drug conjugates

*In vitro* activity tests were carried out on various antibody-drug conjugates of the present invention to detect their cell killing activity.

The testing method was as follows:
The target cells were plated into a 96-well black-bottomed transparent cell culture plate at a certain number. After incubating for 24 hours, samples diluted in a concentration gradient were added. Then, after incubating for 72 hours or 144 hours, a chromogenic reagent was used for color development for 60 minutes, and the plate was read using a microplate reader. The greater the number of living cells, the higher the signal value. Based on the different signal values obtained from different concentration gradient points, a four-parameter fitting curve was formed on the microplate reader, and the C value, that is, the IC₅₀ value, was obtained.

Part of the data were as follows.

**Table 1. Activity data of MCC-AAQ-Exatecan ADCs in the MDA-MB-468 cell line**

| | | | | | | | |
|---|---|---|---|---|---|---|---|
| mAb | Linker Payload | ADC Concentration | HIC-HPLC | SEC-HPLC | | | Activity Data |
| | | mg/mL | DAR | HMW% | Mono% | LMW% | |
| EGFR antibody | MCC-AAQ-Exatecan | 3.0 | 8.0 | 1.0 | 98.7 | 0.3 | IC50 = about 0.082 nM |
| HER2 antibody | MCC-AAN-Exatecan | 3.5 | 8.0 | 1.1 | 98.5 | 0.4 | IC50 = about 0.021 nM |

In addition, the present invention also provides the following Examples.

### Example 10. Preparation of the linker-drug conjugate mc-AAN-Exatecan

### 1. Synthesis of Intermediate 1

After Fmoc-Ala-OH (N-fluorenylmethyloxycarbonyl-L-alanine, CAS No.: 35661-39-3) was activated with HOSu (N-hydroxysuccinimide, CAS No.: 6066-82-6), it was reacted with L-Ala (L-alanine, CAS No.: 56-41-7) to obtain Intermediate 1. Specifically, Fmoc-Ala-OH (3 g, 1.0 eq.) and HOSu (1.45 g, 1.3 eq.) were added into a reaction flask, followed by 21 mL of THF. DCC (2.59 g, 1.3 eq.) was slowly added under stirring while controlling the temperature at room temperature. Then, the reaction was carried out at room temperature. The reaction was monitored by HPLC. After the reaction was completed, the reaction mixture was filtered, and the filter cake was rinsed with THF (6 mL). Purified water (15 mL) was added into the filtrate, followed by L-Ala (1.12 g, 1.3 eq.) and solid sodium bicarbonate (0.81 g, 1.0 eq.), and the mixture was stirred and reacted at room temperature. The reaction was monitored by HPLC. After the reaction was completed, citric acid (2.02 g, 1.0 eq.) was added, and the mixture was stirred. Then, the reaction mixture was extracted with ethyl acetate. The organic phase was concentrated, and DMF (12 mL) was added to dissolve the product. After filtering, pre-HPLC was carried out. After the preparative solution was concentrated until no obvious droplets flowed out, it was extracted with ethyl acetate three times. The ethyl acetate phase was concentrated to dryness to obtain 1.75 g of Intermediate 1, with a reaction yield of 57% for this step. The reaction formula was as shown below:

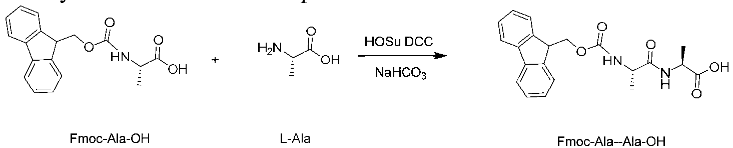

### 2. Synthesis of Intermediate 2

After Intermediate 1 (N-[fluorenylmethoxycarbonyl]-L-alanyl-L-alanine, CAS No.: 87512-31-0) was activated with HOSu (N-hydroxysuccinimide, CAS No.: 6066-82-6), it was reacted with L-Asn (L-asparagine, CAS No.: 70-47-3) to obtain Intermediate 2. Specifically, Intermediate 1 (0.87 g, 1.0 eq.), HOSu (0.34 g, 1.3 eq.), and THF (9 mL) were added into a reaction flask. DCC (0.61 g, 1.3 eq.) was slowly added under stirring while controlling the temperature at room temperature. The reaction was carried out at room temperature. The reaction was monitored by HPLC. After the reaction was completed, the reaction mixture was filtered, and the filter cake was rinsed with THF (2 mL). Purified water (10 mL) was added into the filtrate, followed by L-Asn (0.34 g, 1.1 eq.) and solid sodium bicarbonate (0.19 g, 1.0 eq.), and the mixture was stirred and reacted at room temperature. The reaction was monitored by HPLC. After the reaction was completed, citric acid monohydrate (0.48 g, 1.0 eq.) was added, and the mixture was stirred. The reaction mixture was concentrated to remove most of the solvent, and the residue was subjected to preparation and purification. After the preparative solution was concentrated until no obvious droplets flowed out, it was extracted with ethyl acetate. The organic phase was concentrated to dryness to obtain 826 mg of Intermediate 2, with a reaction yield of 73% for this step. The reaction formula was as shown below:

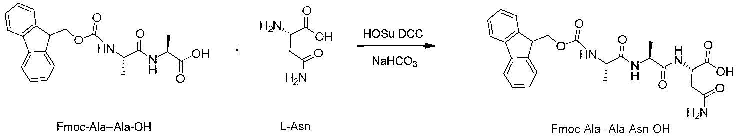

### 3. Synthesis of Intermediate 3

After the Fmoc group was removed from Intermediate 2 by adding DEA (diethylamine, CAS No.: 109-89-7), Intermediate 3 was obtained. Specifically, Intermediate 2 (100 mg) and DMF (1.5 mL) were added into a reaction flask. DEA (300 µL) was added dropwise while controlling the temperature at room temperature. The reaction was carried out at room temperature. The reaction was monitored by HPLC until no Intermediate 2 remained. DMF was removed by concentration. DCM (4 mL) and purified water (4 mL) were added. After stirring, liquid separation was carried out. The aqueous phase was concentrated to dryness to obtain 92 mg of Intermediate 3, with a reaction yield of 166% for this step (due to including part of the solvent). The reaction formula was as shown below:

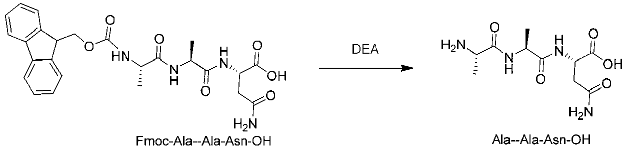

### 4. Synthesis of Intermediate 4

Intermediate 3 was reacted with N-succinimidyl 6-maleimidohexanoate (CAS No.: 55750-63-5) to obtain Intermediate 4. Specifically, Intermediate 3 (92 mg, 1.0 eq.), N-succinimidyl 6-maleimidohexanoate (135 mg, 1.3 eq.), DMF (1.5 mL), and DIPEA (0.059 mL, 1.0 eq.) were added into a reaction flask. The reaction was monitored by HPLC. After the reaction was completed, preparation and purification were carried out. The preparative solution was concentrated to obtain 56 mg of Intermediate 4, with a reaction yield of 35.7% for this step. The reaction formula was as shown below:

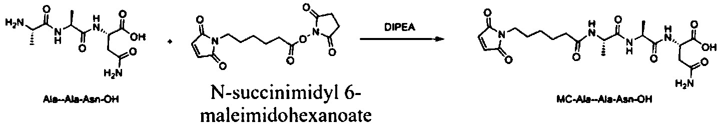

### 5. Synthesis of the product

Intermediate 4 was subjected to a condensation reaction with exatecan mesylate (CAS No.: 169869-90-3) to obtain Intermediate 4. Specifically, Intermediate 4 (26 mg, 1.0 eq.) was added into a reaction flask at room temperature, followed by DMF (1.5 mL). Exatecan mesylate (29.6 mg, 1.0 eq.), EEDQ (20.7 mg, 1.5 eq.), HATU (31.8 mg, 1.5 eq.), DMAP (0.7 mg, 0.1 eq.), and DIPEA (29.2 µL, 3.0 eq.) were added successively, and the reaction was carried out at room temperature. The reaction was monitored by HPLC. After the reaction was completed, preparation and purification were carried out. The preparative solution was concentrated to obtain 16.5 mg of the product (the NMR spectrum was as follows), with a reaction yield of 33.5% for this step. The reaction formula was as shown below:

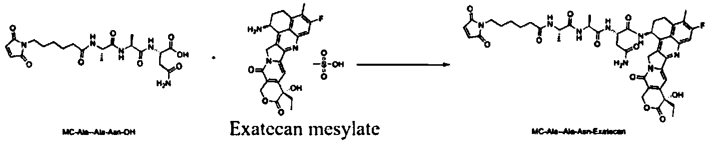

¹H NMR (400 MHz, DMSO-*d*₆) δ 8.22-8.29 (d, *J* =2.0 Hz, 1H), 7.94-8.05 (d, *J* =2.0Hz, 3H), 7.83-7.88(d, *J* =2.0 Hz,1H), 7.75-7.82(d, *J* =2.0 Hz, 1H), 7.34-7.40 (m,1H), 7.28-7.33 (m,1H), 6.96-7.02(d, *J* =8.0 Hz,2H), 6.87-6.94(m,1H), 5.42-5.55(m,1H), 5.41-5.46(m,2H), 5.21-5.26(m,1H), 4.41-4.49(m,1H), 4.00-4.12(m,2H), 3.32-3.40(m,2H), 3.12-3.17(m,2H), 2.86-2.96(m,2H), 2.71-2.76(m,2H), 2.36-2.43(m,2H), 2.14-2.24(m,1H), 1.97-2.07(m,3H), 1.81-1.93(m,2H), 1.36-1.52(m,4H), 1.05-1.25(m,8H), 0.83-0.93(m,3H).

### Example 11. Preparation of the linker-drug conjugate mc-Ala-Ala-Gln-Exatecan

### Synthesis of Intermediate 1, Fmoc-Gln-Exatecan

Fmoc-Gln-OH (N-fluorenylmethyloxycarbonyl-L-glutamine) (76.3 mg, 1.1 eq.) and Exatecan (100 mg, 1.0 eq.) were added into a reaction flask, followed by DMF (1 ml), DIEA (29 mg, 1.5 eq.), and TBTU (72.5 mg, 1.1 eq.), and the reaction was carried out at room temperature. The reaction was monitored by HPLC until no starting materials remained. After the reaction was completed, silica gel was added and thoroughly mixed with the sample. The mixture was concentrated, followed by purification through column chromatography. The product was collected and concentrated to dryness to obtain 140 mg of the product, with a purity of 95% (due to approximately 4% of the by-product Hobt from TBTU that had not been completely removed), and a yield of 94%.

The reaction formula was as shown below:

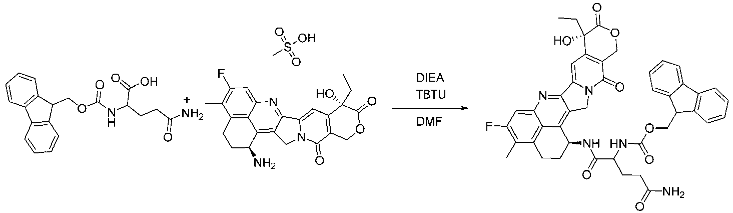

### 1. Synthesis of Intermediate 2, Gln-Exatecan

Intermediate 1, Fmoc-Gln-Exatecan (130 mg, 1.0 eq.) was added into a reaction flask, followed by DCM (2 ml). The mixture was stirred at room temperature, and failed to be dissolved and clarified. DEA (0.5 ml) was added, and the reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. After filtering, an off-white solid was obtained and dried to obtain 95 mg, with a purity of 96%, and a yield of 94%.

The reaction formula was as shown below:

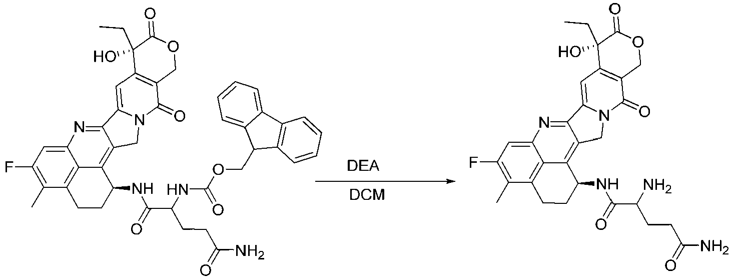

### 2. Synthesis of Intermediate 3, Fmoc-Ala-Ala-Gln-Exatecan

Intermediate 2, Gln-Exatecan (100 mg, 1.0 eq.) was added into a reaction flask, followed by DMF (1 mL), Fmoc-Ala-Ala-OH (67.8 mg, 1.0 eq.), TBTU (68.4 mg, 1.2 eq.), and DIEA (34.4 mg, 1.5 eq.), and the reaction was carried out at room temperature. The reaction was monitored by HPLC until no Intermediate 2 remained, and the reaction was stopped. Silica gel was added and thoroughly mixed with the sample. Purification was carried out by column chromatography. The product was collected to obtain 140 mg of the product, with a purity of 95%, and a yield of 85%.

The reaction formula was as shown below:

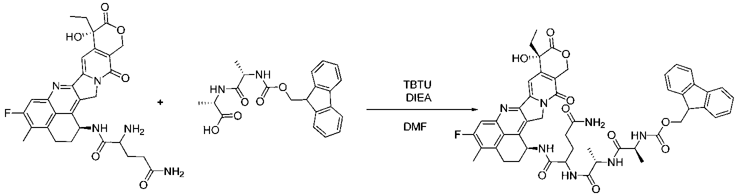

### 3. Synthesis of Intermediate 4, Ala-Ala-Gln-Exatecan

Intermediate 3, Fmoc-Ala-Ala-Gln-Exatecan (140 mg, 1.0 eq.) was added into a reaction flask, followed by DCM (2 ml) and DEA (0.5 ml), and the reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. After filtering, an off-white solid was obtained and dried to obtain 70 mg of the product, with a purity of 96%, and a yield of 66%.

The reaction formula was as shown below:

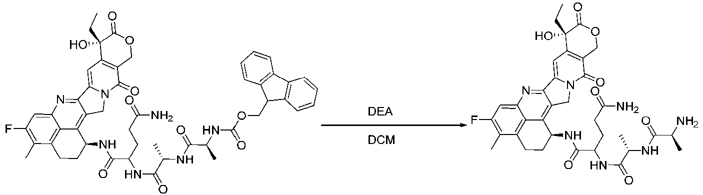

### 4. Synthesis of the product, mc-Ala-Ala-Gln-Exatecan

Intermediate 4, Ala-Ala-Gln-Exatecan (50 mg, 1.0 eq.) was added into a reaction flask, followed by DMF (1 mL), N-succinimidyl 6-maleimidohexanoate (21.8 mg, 1.1 eq.) and DIEA (12.1 mg, 1.5 eq.), and the reaction was carried out at room temperature. The reaction was monitored by HPLC until no Intermediate 4 remained, and the reaction was stopped. Preparation and purification were carried out. The product was collected and concentrated to dryness to obtain 35 mg of the product (the NMR spectrum was as follows), with a purity of 98%, and a yield of 55%.

The reaction formula was as shown below:

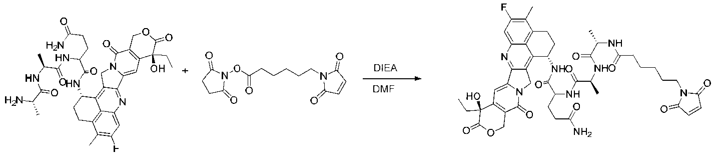

¹H NMR (400 MHz, DMSO-*d*₆) δ 8.28-8.36 (d, *J* =2.0 Hz, 1H), 7.90-7.96(d, *J* =2.0Hz, 2H), 7.75-7.87(d, *J* =2.0 Hz,2H), 7.27-7.33(d, *J* =2.0 Hz, 1H), 7.20-7.25 (m,1H), 6.97-7.03(d, *J* =8.0 Hz,2H), 6.69-6.75(m,1H), 6.49-6.53(m,1H), 5.46-5.55(m,1H), 5.40-5.45(m,2H), 5.24-5.34(m,1H), 5.04-5.14(m,1H), 4.31-4.38(m,1H), 4.02-4.23(m,2H), 3.86-3.96(m,1H), 3.40-3.49(m,1H), 3.33-3.40(m,2H), 3.12-3.20(m,1H), 2.36-2.42(m,2H), 2.15-2.25(m,1H), 2.00-2.12(m,4H), 1.71-1.93(m,3H), 1.36-1.52(m,4H), 1.01-1.20(m,10H), 0.83-0.92(m,3H).

### Example 12. Preparation of the linker-drug conjugate mc-Pro-Exatecan

### 1. Synthesis of Intermediate 1, Fmoc-Pro-Exatecan

Fmoc-Pro-OH (N-fluorenylmethyloxycarbonyl-L-proline) (41 mg, 1.3 eq.) and Exatecan (50 mg, 1.0 eq.) were added into a reaction flask. DMF (1 ml) and DIEA (49 mg, 4 eq.) were added to dissolve the substances. HATU (46 mg, 1.3 eq.) and HOBt (12.7 mg, 1.0 eq.) were added, and the reaction was carried out at room temperature. The reaction was monitored by HPLC until no starting materials remained. After the reaction was completed, silica gel was added and thoroughly mixed with the sample. The mixture was concentrated, followed by purification through column chromatography. The product was collected and concentrated to dryness to obtain 50 mg of the product, with a purity of 95%, and a yield of 73%.

The reaction formula was as shown below:

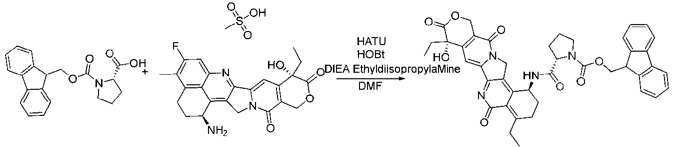

### 2. Synthesis of Intermediate 2, Pro-Exatecan

Intermediate 1, Fmoc-Pro-Exatecan (50 mg, 1.0 eq.) was added into a reaction flask, followed by DCM (1 ml) and DEA (0.2 ml), and the reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. After filtering, an off-white solid was obtained and dried to obtain 30 mg, with a purity of 96%, and a yield of 86%.

The reaction formula was as shown below:

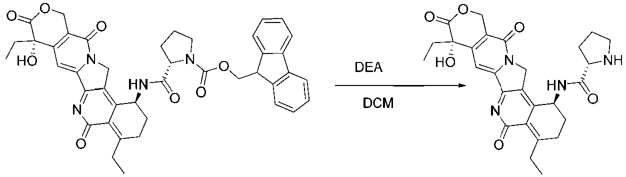

### 3. Synthesis of the product, mc-Pro-Exatecan

Intermediate 2, Pro-Exatecan (30 mg, 1.0 eq.) was added into a reaction flask, followed by DMF (1 mL), N-succinimidyl 6-maleimidohexanoate (21.2 mg, 1.1 eq.) and DIEA (12.1 mg, 1.5 eq.), and the reaction was carried out at room temperature. The reaction was monitored by HPLC until no Intermediate 2 remained, and the reaction was stopped. Silica gel was added and thoroughly mixed with the sample. Purification was carried out by column chromatography. The product was collected to obtain 25 mg of the product (the NMR spectrum was as follows), with a purity of 96%, and a yield of 56%.

The reaction formula was as shown below:

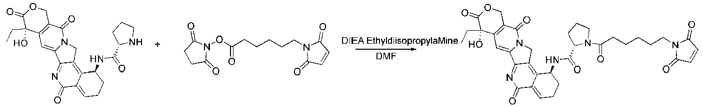

¹H NMR (400 MHz, DMSO-*d*₆) δ 8.17-8.26 (d, *J* =2.0 Hz, 1H), 7.80-7.90 (d, *J* =2.0 Hz, 2H), 7.21-7.28(d, *J* =8.0 Hz,1H), 6.98-7.04(m,1H), 5.01-5.11(m,1H), 4.14-4.24(m,1H), 3.97-4.08(m,1H), 3.47-3.57(m,1H), 3.20-3.27(m,1H), 3.05-3.16(m,2H), 2.30-2.36(m,2H), 2.00-2.15(m,4H), 1.77-1.92(m,3H), 1.65-2.00(m,6H), 1.45-1.64(m,4H), 1.09-1.24(m,4H), 0.95-1.07(m,2H), 0.79-0.94(m,3H).

### Example 13. Preparation of the antibody conjugate HER2 antibody-mc-AAN-Exatecan (DAR8)

1. 10 mg of a HER2 antibody was taken and diluted with PBS buffer to a concentration of 5 mg/mL. The pH of the antibody was adjusted to around 7.5 with a Tris-EDTA solution. The protein concentration was detected using Nanodrop, the net weight of the antibody solution was weighed, and the total protein amount was calculated. 8 folds by number of moles of a TCEP solution was added into the antibody, and the mixture was placed on a 3D shaker and reacted at room temperature for more than 120 min with continuous mixing.
2. An excess of mc-AAN-Exatecan solution (dissolved in DMSO) was added into the reduced antibody solution. After being mixed evenly, the mixture was placed on a 3D shaker and reacted at room temperature for more than 30 min with continuous mixing. After the reaction was completed, an excess of N-acetylcysteine solution was added into the reaction mixture. The mixture was placed on a 3D shaker and reacted at room temperature for more than 30 min with continuous mixing.
3. The conjugated product was purified using a 15 mL 30KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody-drug conjugate, HER2 antibody-mc-AAN-Exatecan, which was stored at 4°C.
4. The protein concentration of the antibody-drug conjugate was detected by the UV/BCA method, the DAR was detected by HIC, and the purity was detected by SEC.

### Example 14. In vitro activity testing of various antibody-drug conjugates

Referring to the process of Example 13, the linker-drug conjugate mc-AAN-Exatecan was conjugated to a HER2 antibody, an EGFR antibody, and a non-specific binding antibody (Anti-HEL) respectively to prepare the corresponding ADCs. The linker-drug conjugate mc-Pro-Exa was conjugated to a HER2 antibody to prepare an ADC. The cell killing activity was detected.

The testing method was as follows:
The target cells were plated into a 96-well black-bottomed transparent cell culture plate at a certain number. After incubating for 24 hours, samples diluted in a concentration gradient were added. Then, after incubating for 72 hours, a chromogenic reagent was used for color development for 60 minutes, and the plate was read using a microplate reader. The greater the number of living cells, the higher the signal value. Based on the different signal values obtained from different concentration gradient points, a four-parameter fitting curve was formed on the microplate reader, and the C value, that is, the IC₅₀ value, was obtained.

Detailed data were as follows:

**Table 2. Activity data of mc-AAN-Exatecan ADCs in different cell lines**

| mAb | Linker Payload | ADC Concentration | HIC-HPLC | SEC-HPLC | | | Activity Data |
|---|---|---|---|---|---|---|---|
| | | mg/mL | DAR | HMW% | Mono% | LMW% | |
| HER2 antibody | mc-AAN-Exatecan | 5.2 | 8.0 | 0.98 | 98.7 | 0.34 | SKBR3 (breast cancer cell line) |
| | | | | | | | mc-AAN-Exatecan ADC: IC50 = 0.051 nM |
| | | | | | | | |
| Anti-HEL antibody (non-binding) | | 2.7 | 8.0 | 1.69 | 98.10 | 0.25 | No specific activity |
| EGFR antibody | | 3.1 | 8.0 | 1.86 | 96.73 | 1.4 | DIFI (colorectal cancer cell line) mc-AAN-Exatecan ADC: IC50 = about 0.045 nM |

**Table 3. Activity data of Exatecan ADC vs Payload**

| Sample Name | Linker Payload | ADC Concentration | HIC-HPLC | SEC-HPLC | | | Activity Data |
|---|---|---|---|---|---|---|---|
| | | mg/mL | DAR | HMW% | Mono% | LMW% | |
| | | | | | | | SKBR3 (breast cancer cell line) |
| HER2 antibody | mc-AAN-Exatecan | 5.2 | 8.0 | 0.98 | 98.7 | 0.34 | 1. mc-AAN-Exatecan ADC: IC50 = 0.051 nM |
| | | | | | | | 2. Extecan: IC50 = 2.021 nM |

**Table 4. Activity data of mc-Pro-Exa ADC in the breast cancer cell line**

| mAb | Linker Payload | ADC Concentration | HIC-HPLC | SEC-HPLC | | | Activity Data |
|---|---|---|---|---|---|---|---|
| | | mg/mL | DAR | HMW% | Mono% | LMW% | |
| HER2 antibody | me-Pro-Exa | 3.7 | 8.0 | 0.94 | 98.7 | 0.09 | SKBR3 (breast cancer cell line: IC50 = 0.087 nM) |

### Example 15. Preparation of ADCs using linkers or cytotoxins with different structures and detection and analysis

Referring to the preparation processes of the Linker-Payload in Example 10 and the ADC stock solution in Example 13, the inventors prepared the following ADC stock solutions, and detected and analyzed the basic quality characteristics of the obtained ADC stock solutions.

After conjugation to the same HER2 antibody, the DAR values were all close to 8. Based on the analysis of the detection data of these ADCs, the following conclusions could be obtained:
1. The polymer content (HMW%) of the ADCs prepared from the Linker-Payload without PAB was found to be significantly lower than that of the ADCs prepared from the Linker-Payload containing the PAB fragment, indicating that the PAB fragment was not conducive to the preparation of ADCs from this type of Linker-Payload. The specific data were as shown in Table 5.
2. The Payload containing a primary amine (-NH₂) structure could be released through a Linker without the PAB fragment, while the Payload containing a non-primary amine group (the -NH-(i-Pr) structure) could not be released through a Linker without the PAB fragment. The specific data were as shown in Table 6.

The inventors investigated the polymer content in ADCs obtained by conjugating mc-Pro-PAB-Exatecan to the antibody TF (i.e., TF-mAb-H39 in CN 201610705557.4) and mc-Pro-Exatecan to a HER2 antibody, and found that the influence of the PAB structure was very significant. After PAB was removed from the Linker, the polymer content could be reduced from approximately 70-80% to 1%.

**Table 5. Analysis data of the influence of the PAB fragment on the residual amount of HMW%**

| mAb | Linker Payload | ADC Concentration | HIC-HPLC | SEC-HPLC | | |
|---|---|---|---|---|---|---|
| | | mg/mL | DAR | HMW% | Mono% | LMW% |
| HER2 antibody | mc-Pro-Exatecan | 4.6 | 8.0 | 1.07 | 98.6 | 0.38 |
| TF antibody | mc-Pro-PAB-Exatecan | 4.4 | 7.1 (estimated value) | 76.8 | 23.2 | 0.03 |

When the *in vitro* cell killing activity of ADCs obtained by conjugating mc-Pro-Belotecan and mc-Pro-Exatecan to a HER2 antibody respectively was investigated, it was found that the ADC conjugated to mc-Pro-Belotecan had no tumor cell killing activity, indicating that its structure was stable and the Payload could not be released by means of an enzymatic hydrolysis reaction. In contrast, the ADC conjugated to mc-Pro-Exatecan showed good killing activity, suggesting that this structure could release the Payload. Moreover, the ADC produced by conjugating mc-Pro-Exatecan to a non-binding antibody (without targeted endocytosis) had no specific cell killing activity, suggesting that this structure was stable extracellularly and the Payload could only be released after the structure was endocytosed into the cell. In addition, by comparing the chemical structures of mc-Pro-Belotecan and mc-Pro-Exatecan, it was found that the key difference between the two was that Belotecan was linked to Proline via a -NH-(i-Pr) structure, while Exatecan was linked to Proline via a -NH₂ structure. Therefore, it was presumed that if the amino acid fragment in the Linker was linked to a Payload containing a primary amine (-NH₂) structure, the Payload could be released by means of an enzymatic hydrolysis reaction in the case of removing the hydrophobic structure PAB (for most of the ADCs currently under research, the PAB structure, as a self-releasing fragment, is an essential structure for the effective release of the Payload), which could not only reduce the hydrophobicity of the Linker-Drug and reduce the content of the polymers produced in the preparation of the ADC, but also could exhibit the corresponding cell killing activity.

**Table 6. Analysis data of the conjugation of different Linker-Payloads to a HER2 antibody respectively**

| mAb | Linker Payload | ADC Concentration | HIC-HPLC | SEC-HPLC | | | Activity Data |
|---|---|---|---|---|---|---|---|
| | | mg/mL | DAR | HMW% | Mono% | LMW% | |
| HER2 antibody | mc-Pro-Belotecan | 4.5 | 8.0 | 1.1 | 98.7 | 0.20 | No specific activity |
| | mc-Pro-Exatecan | 4.6 | 8.0 | 1.07 | 98.6 | 0.38 | SKBR3 (breast cancer cell line) |
| | | | | | | | mc-AAN-Exatecan ADC: IC50 = 0.087 nM |

Although the specific embodiments of the present invention have been described in detail, it will be understood by those skilled in the art that: according to all the teachings disclosed, various modifications and replacements can be made to those details, and these changes are all within the scope of protection of the present invention. The full scope of the present invention is given by the appended claims and any equivalents thereof.

## Claims

1. A linker-drug conjugate, or a pharmaceutically acceptable salt or solvate thereof, or a solvate of the salt, **characterized in that** the linker-drug conjugate has a structure as shown in formula I:
M-X-D formula I
wherein,
M is a chemical structure comprising a maleimide (m) fragment or a cyclooctyne fragment;
X is a linker fragment consisting of 1-5 amino acids or their derivatives, preferably selected from Ala-Ala-Ala-Ala-Asn, Ala-Ala-Ala-Asn, Ala-Ala-Asn, Ala-Asn, Asn, Ala-Ala-Ala-Ala-(3-cyano-alanine), Ala-Ala-Ala-(3-cyano-alanine), Ala-Ala-(3-cyano-alanine), Ala-(3-cyano-alanine), 3-cyano-alanine, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Ala-Gln, Ala-Ala-Ala-Gln, Ala-Ala-Gln, Ala-Gln, Ala-Ala-Ala-Ala-(2-amino-4-cyanobutanoic acid), Ala-Ala-Ala-(2-amino-4-cyanobutanoic acid), Ala-Ala-(2-amino-4-cyanobutanoic acid), Ala-(2-amino-4-cyanobutanoic acid), 2-amino-4-cyanobutanoic acid, Gly-Gly-Gly-Gln, Gly-Gly-Gln, Gly-Gln, Gln, and their deuterated compounds; and
D is a cytotoxin.

2. The linker-drug conjugate, or the pharmaceutically acceptable salt or solvate thereof, or the solvate of the salt of claim 1, **characterized in that** the chemical structure comprising a maleimide (m) fragment is selected from 4-(N-maleimidomethyl)-cyclohexane-1-formyl (MCC), 6-maleimidocaproyl (MC), M-(PEG)ₙ, MC-(PEG)ₙ, and MCC-(PEG)ₙ, the structures of which are as shown below:
| | |
|---|---|
| MCC | 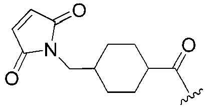 |
| MC | 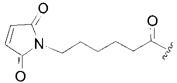 |
| M-(PEG)ₙ | 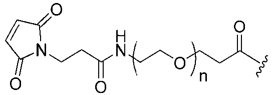 |
| MC-(PEG)ₙ | 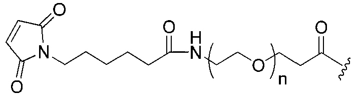 |
| MCC-(PEG)ₙ | 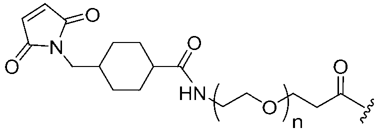 |
| | |
|---|---|
| wherein, n represents the degree of polymerization of PEG, and is preferably an integer between 2 and 12. | |

3. The linker-drug conjugate, or the pharmaceutically acceptable salt or solvate thereof, or the solvate of the salt of claim 1, **characterized in that** the chemical structure comprising a cyclooctyne fragment is selected from BCN, DIBO, DIBAC, BARAC, etc., the structures of which are as shown below:
| | |
|---|---|
| BCN | 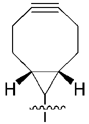 |
| DIBO | 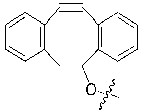 |
| DIBAC | 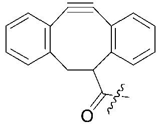 |
| BARAC | 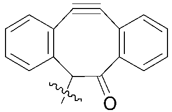 |

4. The linker-drug conjugate, or the pharmaceutically acceptable salt or solvate thereof, or the solvate of the salt of any one of claims 1-3, **characterized in that** the linker fragment consisting of 1-5 amino acids or their derivatives is selected from Ala-Ala-Ala-Ala-Asn, Ala-Ala-Ala-Asn, Ala-Ala-Asn, Ala-Asn, Asn, Ala-Ala-Ala-Ala-(3-cyano-alanine), Ala-Ala-Ala-(3-cyano-alanine), Ala-Ala-(3-cyano-alanine), Ala-(3-cyano-alanine), 3-cyano-alanine, Ala-Ala-Ala-Pro, Ala-Ala-Pro, Ala-Pro, Pro, Pro-Asn, Asn-Pro, Lys, Lys-Asn, Lys-Pro, Ala-Ala-Ala-Ala-Gln, Ala-Ala-Ala-Gln, Ala-Ala-Gln, Ala-Gln, Ala-Ala-Ala-Ala-(2-amino-4-cyanobutanoic acid), Ala-Ala-Ala-(2-amino-4-cyanobutanoic acid), Ala-Ala-(2-amino-4-cyanobutanoic acid), Ala-(2-amino-4-cyanobutanoic acid), 2-amino-4-cyanobutanoic acid, Gly-Gly-Gly-Gln, Gly-Gly-Gln, Gly-Gln, Gln, and their deuterated compounds.

5. The linker-drug conjugate, or the pharmaceutically acceptable salt or solvate thereof, or the solvate of the salt of any one of claims 1 to 4, **characterized in that** D is a cytotoxin containing a hydroxyl (-OH), primary amine (-NH₂), or secondary amine (-NHR) group;
preferably, the cytotoxin containing a hydroxyl (-OH), primary amine (-NH₂), or secondary amine (-NHR) group is selected from camptothecin topoisomerase I inhibitors, Daunorubicin topoisomerase II inhibitors, calendulin derivatives, molecular glue compounds, and immunosuppressants;
more preferably, the cytotoxin containing a hydroxyl (-OH), primary amine (-NH₂), or secondary amine (-NHR) group is selected from Exatecan, 14-aminocamptothecin (14-AC), 9-aminocamptothecin (9-AC), belotecan, MMAE and its derivatives, MMAF and its derivatives, lenalidomide, and Rapamycin and its derivatives; and
most preferably, the cytotoxin containing a hydroxyl (-OH), primary amine (-NH₂), or secondary amine (-NHR) group is selected from Exatecan, belotecan, and Rapamycin.

6. The linker-drug conjugate, or the pharmaceutically acceptable salt or solvate thereof, or the solvate of the salt of any one of claims 1 to 5, **characterized in that** the linker-drug conjugate is MCC-AAQ-Exatecan, which has the structure as shown below:

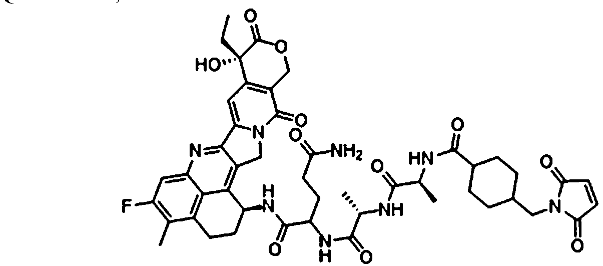

7. An antibody-drug conjugate, or a pharmaceutically acceptable salt or solvate thereof, or a solvate of the salt, **characterized in that** the antibody-drug conjugate is formed by conjugating the linker-drug conjugate of any one of claims 1-6 to an antibody;
preferably, the antibody-drug conjugate has a structure as shown in formula II:
Ab-(L-D)ₚ formula II
wherein,
Ab is an antibody;
L is a linker, with a structure of M'-X, wherein, M' is linked to Ab, and X is linked to D;
M' is a chemical structure formed by linking M as defined in any one of claims 1-6 to Ab through a maleimide group;
X is a linker fragment consisting of 1-5 amino acids or their derivatives as defined in any one of claims 1-6;
D is a cytotoxin as defined in any one of claims 1-6; and
p is any numerical value between 2 and 8.

8. The antibody-drug conjugate, or the pharmaceutically acceptable salt or solvate thereof, or the solvate of the salt of claim 7, **characterized in that** the antibody is selected from murine antibodies, rabbit antibodies, phage display-derived antibodies, yeast display-derived antibodies, chimeric antibodies, humanized antibodies, fully human antibodies, antibody fragments, bispecific antibodies, and multispecific antibodies; and
preferably, the antibody is a monoclonal antibody, selected from: HER2 antibodies and EGFR antibodies.

9. A pharmaceutical composition comprising the linker-drug conjugate, or the pharmaceutically acceptable salt or solvate thereof, or the solvate of the salt of any one of claims 1-6, or the antibody-drug conjugate, or the pharmaceutically acceptable salt or solvate thereof, or the solvate of the salt of claim 6 or 7; wherein
preferably, the pharmaceutical composition further comprises at least one of a chemotherapeutic drug, an immunotherapeutic drug, and an immunosuppressant for treating a tumor; and
preferably, the pharmaceutical composition further comprises at least one pharmaceutical auxiliary material.

10. Use of the linker-drug conjugate, or the pharmaceutically acceptable salt or solvate thereof, or the solvate of the salt of any one of claims 1-6, or the antibody-drug conjugate, or the pharmaceutically acceptable salt or solvate thereof, or the solvate of the salt of claim 7 or 8 in the preparation of a medicament for the prevention and/or treatment of a tumor.

11. A method for preparing the linker-drug conjugate, or the pharmaceutically acceptable salt or solvate thereof, or the solvate of the salt of any one of claims 1-6, comprising the following steps:
1. preparing a linker fragment X consisting of 1-5 amino acids or their derivatives;
2. linking X to a chemical structure M comprising a maleimide (m) fragment or a cyclooctyne fragment to obtain M-X; and
3. linking a cytotoxin D to M-X to obtain the linker-drug conjugate M-X-D.

12. The method of claim 11, **characterized in that** the linker-drug conjugate is MCC-AAQ-Exatecan, and the method comprises the following steps:
1. reacting Fmoc-Gln with Exatecan to obtain Intermediate 1, wherein, the structural formula of Intermediate 1 is as shown below: 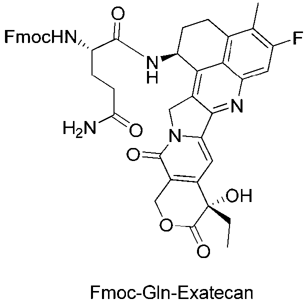
2. removing the Fmoc protecting group from Intermediate 1 using DEA to obtain Intermediate 2, wherein, the structural formula of Intermediate 2 is as shown below: 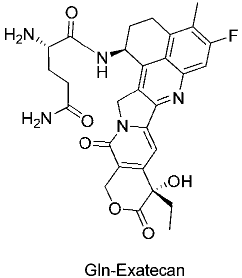
3. reacting Intermediate 2 with Fmoc-Ala-Ala to obtain Intermediate 3, wherein, the structural formula of Intermediate 3 is as shown below: 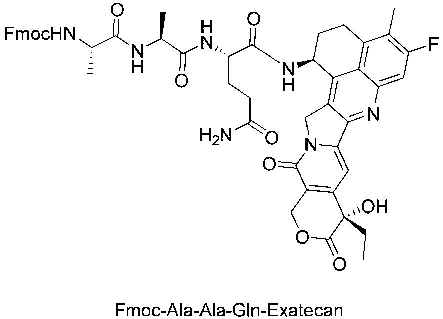
4. removing the Fmoc protecting group from Intermediate 3 to obtain Intermediate 4, wherein, the structural formula of Intermediate 4 is as shown below: 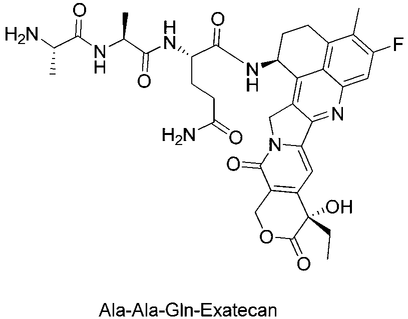
5. and condensing Intermediate 4 with MCC to obtain the product, wherein, the structural formula of the product is as shown below:
